(19) Europäisches Patentamt
European Patent Office
Office européen des brevets

(11) Publication number: **0 090 603**
**A2**

(12) **EUROPEAN PATENT APPLICATION**

(21) Application number: 83301654.6

(22) Date of filing: 24.03.83

(51) Int. Cl.³: C 07 D 407/06, A 61 K 31/35

(30) Priority: 25.03.82 GB 8208816

(43) Date of publication of application: 05.10.83
Bulletin 83/40

(84) Designated Contracting States: CH DE FR GB IT LI NL

(71) Applicant: BEECHAM GROUP PLC, Beecham House
Great West Road, Brentford Middlesex (GB)

(72) Inventor: Crimmin, Michael John, 60 Corsletts Avenue,
Broadbridge Heath Horsham Sussex (GB)
Inventor: Rogers, Norman Harold, 22 Comptons Lane,
Horsham Sussex (GB)

(74) Representative: Cresswell, Thomas Anthony et al,
European Patent Attorney Beecham Pharmaceuticals
Great Burgh Yew Tree Bottom Road, Epsom Surrey
KT18 5XQ (GB)

(54) **Antibacterial compounds.**

(57) Acids of formula (I):

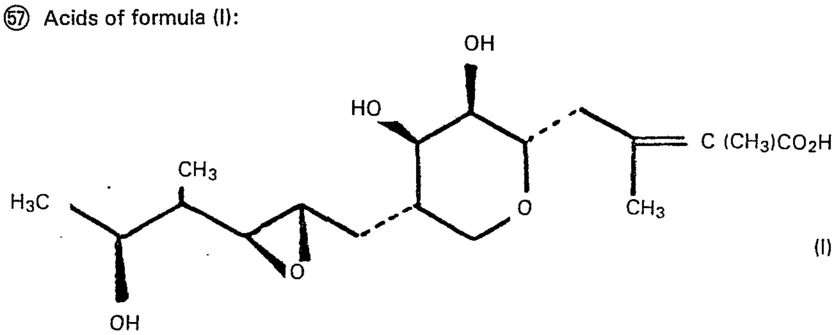

(I)

and salts and esters thereof are useful as intermediates in producing the esters. The esters have antibacterial and/or anti-mycoplasmal activity.

EP 0 090 603 A2

## ANTIBACTERIAL COMPOUNDS

The present invention relates to a class of compounds having antibacterial and/or antimycoplasmal activity, to processes for their production and to their use in human and veterinary medicine. The invention also extends to intermediates useful in the production of such compounds.

According to the present invention there is provided an acid of formula (I):

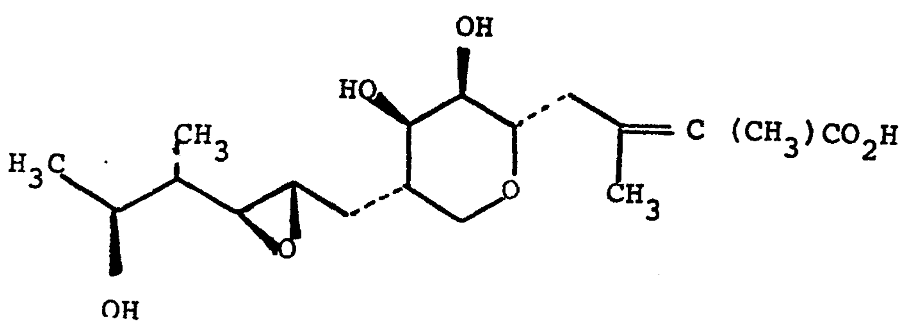

(I)

or a salt or ester thereof.

Suitable salts of the acids of formula (I) include aluminium, alkaline earth metal, alkali metal, ammonium and substituted ammonium salts, especially sodium and potassium salts.

Suitable esters of the acids of formula (I) include optionally substituted alkyl, cycloalkyl, alkenyl, alkynyl and aralkyl esters, especially optionally substituted alkyl and optionally substituted phenylalkyl esters.

All the compounds of formula (I) are useful as chemical intermediates in producing esters of formula (I). Moreover, the esters of formula (I) have antibacterial and/or antimycoplasmal activity and are, therefore, useful in treating infections of human or non-human animals.

Accordingly, in a particular aspect, the present invention provides an ester of formula (IA):

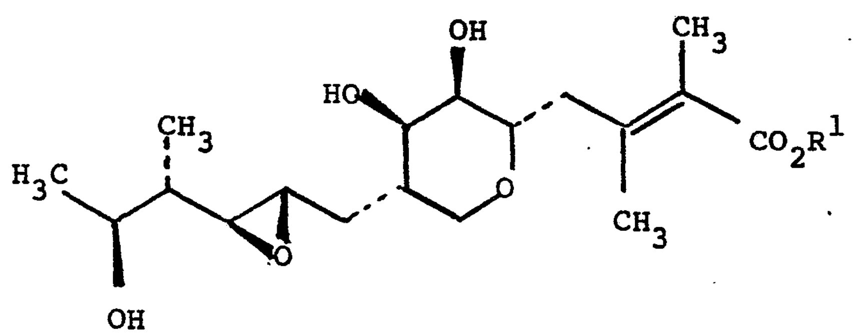

(IA)

wherein $R^1$ is $C_{1-20}$ alkyl, $C_{3-7}$ cycloalkyl, $C_{2-8}$ alkenyl, $C_{2-8}$ alkynyl each of which may be optionally substituted.

Suitable substituents for alkyl, cycloalkyl, alkenyl and alkynyl groups include halogen, carboxy, $C_{1-6}$ alkoxycarbonyl, carbamoyl, mono- or di-($C_{1-6}$)alkylcarbamoyl, sulphamoyl, mono- and

di-($C_{1-6}$)alkylsulphamoyl, amino, mono- and di-($C_{1-6}$)alkylamino, $C_{1-6}$ acylamino, ureido, $C_{1-6}$ alkoxycarbonylamino, 2,2,2-trichloroethoxycarbonylamino, optionally substituted phenyl, heterocyclyl, hydroxy, $C_{1-6}$ alkoxy, oxo, aroyl, 2-thenoyl, $C_{1-6}$ alkylthio, $C_{1-6}$ alkanesulphinyl, $C_{1-6}$ alkanesulphonyl, hydroxyimino, hydrazono, benzohydroximoyl, 2-thiophenecarbohydroximoyl.

Suitable substituents for phenyl groups include halogen, $C_{1-6}$ alkyl, $C_{1-6}$ alkoxy, hydroxy, carboxy, $C_{1-6}$ alkoxycarbonyl, carbamoyl, mono- or di-($C_{1-6}$)alkylcarbamoyl, sulphamoyl, mono- and di-($C_{1-6}$)sulphamoyl, cyano, nitro, amino, mono- and di-($C_{1-6}$)alkylamino, $C_{1-6}$ acylamino, ureido, $C_{1-6}$ alkoxycarbonylamino, 2,2,2-trichloroethoxycarbonylamino, $C_{1-6}$ alkanoyl, $C_{1-6}$ alkylthio, $C_{1-6}$ alkanesulphinyl and $C_{1-6}$ alkanesulphonyl.

Compounds of formula (I) have a tri-substituted double bond and this may be in either the E or Z configuration giving rise to two geometrically isomeric forms. The present invention encompasses both such isomers individually and admixed in any proportions. In general, in the esters of formula (I), greater biological activity is associated with the E isomer and for this reason the E isomer is preferred. Apart from the configuration of the double bond, the absolute stereochemistry of the compounds is shown in formula (I). For convenience, the structural formulae below do not show absolute configurations but these should be assumed to be the same as in formula (I).

The present invention also provides a process for producing a compound of formula (I) which process comprises:

(a) methylation of a compound of formula (II):

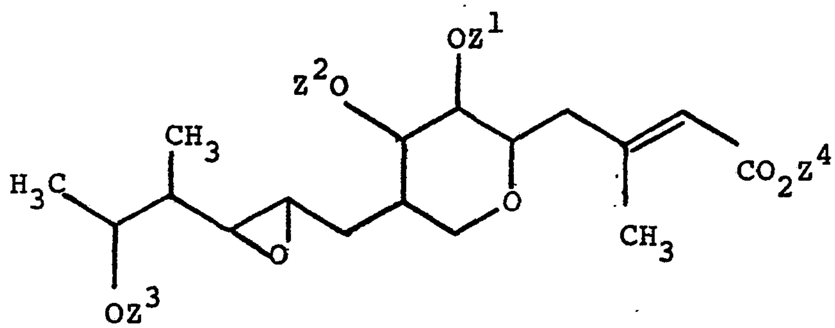

(II)

wherein $Z^1$, $Z^2$ and $Z^3$ are hydrogen or hydroxyl-protecting groups;

and $Z^4$ is hydrogen, a salt-forming cation, or ester-forming radical or a carboxyl-protecting group, including suitable values of $R^1$, especially methyl or ethyl,

or (b) reacting a compound of formula (III):

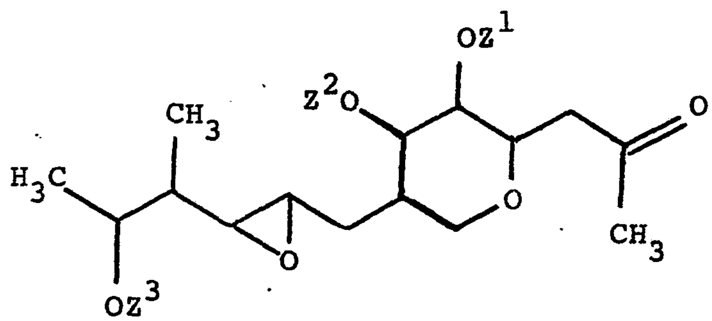

(III)

wherein $Z^1$, $Z^2$ and $Z^3$ are hydroxyl-protecting groups with a compound of formula (IV):

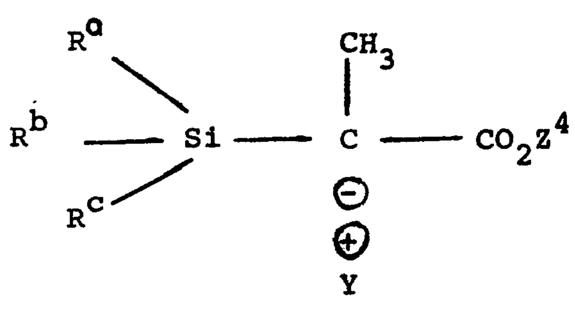

(IV)

wherein $Z^4$ is as defined with respect to formula (II), $R^a$, $R^b$ and $R^c$ are the same or different and each is lower alkyl, aryl or aralkyl; and $Y^+$ is a counter-ion;

or (c) converting a compound of formula (V):

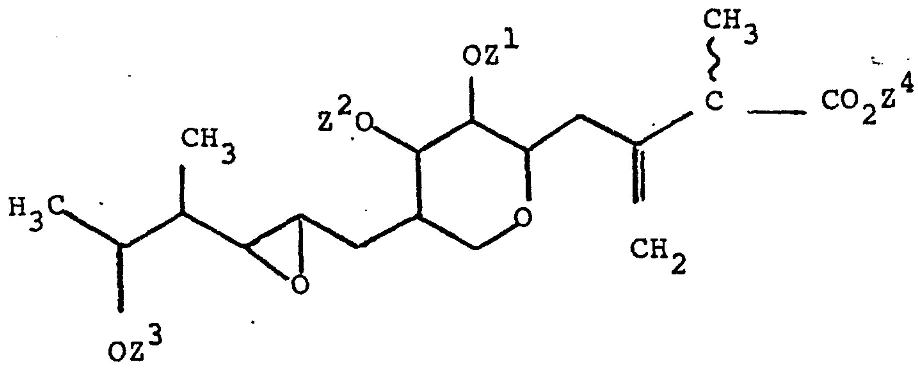

(V)

wherein $Z^4$ is as defined with respect to formula (II), and $Z^1$, $Z^2$ and $Z^3$ are hydrogen or hydroxyl-protecting groups,

into a compound of formula (I) or a protected derivative thereof;

and, if necessary, isomerising a Z isomer so obtained to produce the corresponding E isomer, and, if necessary, removing any hydroxyl-protecting groups and/or carboxyl protecting groups to form the desired compound of formula (I),

and optionally esterifying an acid or salt of formula (I) and de- or trans-esterifying an ester of formula (I).

Methylation of a compound of formula (II) may be achieved by conventional methods. A suitable method is to react the compound of formula (II) with a strong, non-nucleophilic base, such as lithium diisopropylamide in tetrahydrofuran at low temperature, eg -78°C, then react the dienolate with a methylating reagent, such as iodomethane, and finally to re-conjugate the carbon-carbon double bond by treatment with a base,according to Scheme I:

SCHEME I:

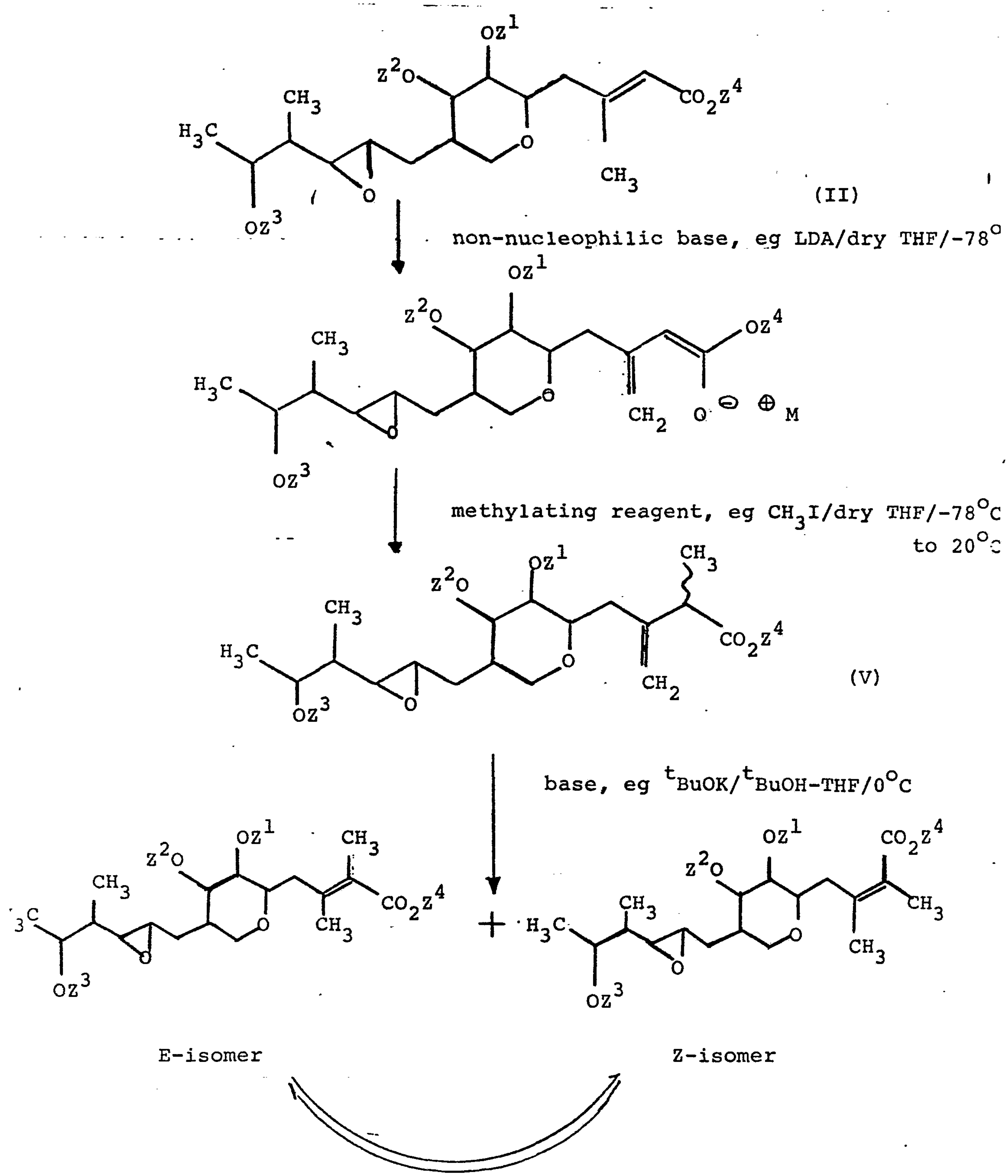


photo isomerisation
eg. low pressure mercury lamp
in benzene soln.

BAD ORIGINAL

It will be appreciated that this method employs as a final step the process variant (c) described above.

The reaction of a compound of formula (III) with a compound of formula (IV) is usually carried out in an inert solvent such as diethyl ether, dimethoxyethane, hexane, benzene or tetrahydrofuran, for example, at a temperature of from about -78°C to about room temperature. Under these conditions the reaction proceeds smoothly over a period of from a few minutes to a few hours and the product may be isolated by any of the usual techniques, e.g. solvent evaporation or anti-solvent precipitation followed by filtration. Purification of the product may be by any of the usual chromatographic or recrystallisation techniques.

The preferred embodiment of this process comprises reacting the compound of formula (III) with a compound of formula (IV). In this case $R^a$, $R^b$ and $R^c$ are, preferably, methyl t-butyl or phenyl and $Y^+$ is preferably an alkali metal ion, especially lithium. Suitably $Z^1$, $Z^2$ and $Z^3$ are silyl groups, especially trimethylsilyl, t-butyldimethylsilyl, phenyldimethylsilyl or diphenylmethylsilyl.

As mentioned above in connection with Scheme I, the conversion of a compound of formula (V) to a compound of formula (I) is suitably effected using a base, especially an alkali metal alkoxide, in a suitable solvent such as potassium t-butoxide in t-butanol-tetrahydrofuran (1:1). Conveniently the alkali metal alkoxide is formed <u>in situ</u> from the corresponding alcohol which also acts as the solvent.

Suitably the alkali metal alkoxides are used at ambient or low temperatures, for instance -78°C to 20°C, preferably at about 0°C. Alternatively amine

bases may be used, suitably at ambient or elevated temperatures, for instance at up to 100°C.

It has been found that the use of an amine base tends to favour formation of the Z-isomer, whereas the alkoxide bases favour formation of the E-isomer.

Isomerisation of the compounds of formula (I), especially in order to produce the E isomer from the Z isomer, may be effected by conventional methods, such as photoisomerisation using a low pressure mercury lamp, for instance as described in J.C.S. Perkin, Trans. I, (1978), 561.

Although each of the above reactions are possible without hydroxyl protection, in general higher yields of the compounds of formula (I) are formed if the hydroxyl groups are protected. Suitable protecting groups include silyl groups since these are readily removed under mild conditions. Such groups are introduced using conventional silylating agents, including halosilanes and silazanes, of the formulae below:

$L_3SiX$
$L_2SiX_2$
$L_3SiNL_2$
$L_3SiNHSiL_3$
$L_3SiNHCOL$
$L_3SiNHCONHSiL_3$
$LNHCONHSiL_3$

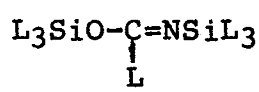

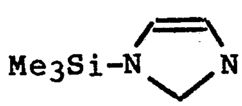

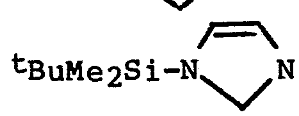

$^tBuMe_2Si-O-SO_2-CF_3$

wherein X is halogen and each group L is independently selected from hydrogen, alkyl, alkoxy, aryl or aralkyl. A preferred silyating agent is trimethylsilyl chloride. Particularly suitable protecting groups are trimethylsilyl, t-butyldimethylsilyl and t-butyldiphenylsilyl groups. Preferred protecting

groups are trimethylsilyl groups because of their ease of removal. These protecting groups are also useful for protecting the carboxyl group of the acids of formulae (II), (IV) and (V), as a group $Z^4$.

When producing the acid of formula (I) from the corresponding ester of formula (I) or when interconverting esters of formula (I) it is desirable to protect the glycol moiety of the ester. This may be effected by forming a cyclic derivative using a compound of formula (VI):

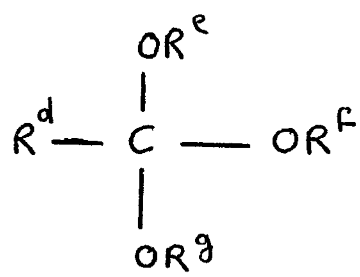

(VI)

wherein $R^d$ is hydrogen or $C_{1-6}$ alkyl and each of $R^e$, $R^f$ and $R^g$ is $C_{1-6}$ alkyl. In the cyclic derivative $Z^1$ and $Z^2$ together are a moiety:

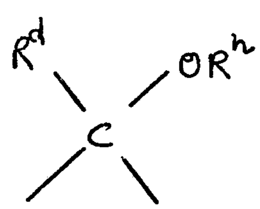

wherein $R^h$ is $C_{1-6}$ alkyl.

Suitably $R^d$ is hydrogen, methyl, ethyl, n- or iso-propyl; most suitably it is hydrogen. The groups $R^e$, $R^f$ and $R^g$ are suitably methyl, ethyl, n- or iso-propyl, n-, iso-, sec- or t-butyl; most suitably methyl.

In each case the protecting groups described above may be removed by mild acid hydrolysis followed by alkaline hydrolysis, for instance as described in J.P. Clayton, K. Luk and N.H. Rogers, J.C.S. Perkin Trans I, (1979), 308.

The compounds of formulae (II) and (III) wherein $Z^1$, $Z^2$ and $Z^3$ are hydrogen and processes for their production, are described in U.K. Patent Nos. 1,587,059 and 1,587,060. Derivatives thereof wherein $Z^1$, $Z^2$ and $Z^3$ are hydroxyl protecting groups may be produced by conventional methods such as those mentioned above. When these compounds are produced with hydroxyl protecting groups already in place they may be used directly or even <u>in situ</u> in the above reactions or may be optionally deprotected and/or isolated.

The acids of formula (I) may be converted into salts by conventional salt forming processes.

The esters of formula (I) may also be prepared by reacting a salt of formula (I) as defined above, with a compound of formula (XI):

$$X—R^1 \qquad (XI)$$

in which $R^1$ is an ester forming group and X is a leaving group such as a halogen atom or an alkyl or aryl sulphonate group, preferably a chlorine or bromine atom or a mesylate group.

The reaction is suitably carried out in a polar aprotic solvent such as N,N-dimethylformamide (DMF) or dimethylacetamide (DMAc).

De-esterification and trans-esterification of an ester of formula (I) may be effected by conventional methods.

Compounds of formula (V) as hereinbefore defined are novel and useful as intermediates in the production of compounds of formula (I).

Accordingly, the present invention provides a compound of formula (V), as hereinbefore defined.

Compounds of formula (V) may be produced by conventional methods from monic acid A or its salts or esters as appropriate as outlined in Scheme I.

The infections against which compounds of this invention are particularly useful include venereal disease. They are also effective in the treatment of respiratory infections such as bacterial bronchitis; and bacterial meningitis, non-specific urethritis and pneumonia. In animals it may be employed for the treatment of mastitis in cattle, for swine dysentery, and for mycoplasma infections in animals such as turkeys, chickens, pigs and cattle.

Some of the human and veterinary diseases either caused by mycoplasma species or in which they play a prominent role, and against which compounds of this invention are effective, are as follows:

Avian

M. gallisepticum - chronic respiratory diseases (air-sacculitis) of chickens and turkeys

Bovine

| | |
|---|---|
| M.bovis | - mastitis, respiratory disease and arthritis of cattle |
| M. dispar | - calf pneumonia |

Porcine

| | |
|---|---|
| M. suipneumoniae | - enzootic pneumonia of pigs |
| M. hyorhinis | - arthritis in pigs |
| M. hyosynoviae | |

Human

| | |
|---|---|
| M. pneumoniae | - primary atypical pneumonia |

Compounds of the present invention are particularly useful in the treatment of enzootic pneumonia in animals such as pigs, cattle and sheep, because they also have activity against the bacteria Bordetella bronchiseptica, Pasteurella multocida and Haemophilus spp, which often cause respiratory complications in cases of this disease.

This invention also provides a pharmaceutical or veterinary composition which comprises a compound of formula (I) (hereinafter referred to as the "drug") together with a pharmaceutically or veterinarily acceptable carrier or excipient.

The compositions may be formulated for administration by any route, and would depend on the disease being treated. The compositions may be in the form of tablets, capsules, powders, granules, lozenges, liquid or gel preparations, such as oral, topical or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinyl-pyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin the drug may be made up into a cream, lotion or ointment. Cream or ointment formulations that may be used for the drug are conventional formulations well known in the art, for example, as described in standard text books of pharmaceutics and cosmetics, such as Harry's Cosmeticology published by Leonard Hill Books, and the British Pharmacopoeia.

Suppositories will contain conventional suppository bases, e.g. cocoa-butters or other glyceride.

For parenteral administration, fluid unit dosage forms are prepared utilizing the drug and a sterile vehicle. The drug, depending on the vehicle and concentration used, can either be suspended or dissolved in the vehicle. In preparing solutions the drug can be dissolved in water for injection and filter sterilized before filling into a suitable vial or ampoule and sealing. Advantageously, adjuvants such as a local anaesthetic, preservative and buffering agents can be dissolved in the vehicle. To enhance the stability the composition can be frozen after filling into the vial and water removed under vacuum. The dry lypophilized powder is then sealed in the vial. Parenteral suspensions are prepared in substantially the same manner except that the drug is suspended in the vehicle instead of being dissolved and sterilisation cannot be accomplished by filtration. The drug can be sterilised by exposure to ethylene oxide before suspending in the sterile vehicle. Advantageously, a surfactant or wetting agent is included in the composition to facilitate uniform distribution of the drug.

For topical application to the ear, the drug may be made up into a solution or suspension in a suitable liquid carrier, such as water, glycerol, diluted ethanol, propylene glycol, polyethylene glycol or fixed oils.

For topical application to the eye, the drug is formulated as a solution or suspension in a suitable, sterile aqueous or non-aqueous vehicle. Additives, for instance buffers such as sodium metabisulphite or

disodium edetate; preservatives including bactericidal and fungicidal agents, such as phenylmercuric acetate or nitrate, benzalkonium chloride or chlorhexidine, and thickening agents such as hypromellose may also be included.

The dosage employed for compositions administered topically will, of course, depend on the size of the area being treated. For the ears and eyes each dose will typically be in the range from 10 to 100 mg of the drug.

Veterinary compositions for intramammary treatment of mammary disorders in animals, especially bovine mastitis, will generally contain a suspension of the drug in an oily vehicle.

The compositions may contain from 0.1% to 99% by weight, preferably from 10-60% by weight, of the drug,depending on the method of administration. Where the compositions comprise dosage units, each unit will preferably contain from 50-500 mg, of the drug. The dosage as employed for adult human treatment will preferably range from 100 mg to 3 g, per day, for instance 250 mg to 2 g, of the drug per day, depending on the route and frequency of administration.

Alternatively, the drug may be administered as part of the total dietary intake. In this case the amount of drug employed may be less than 1% by weight of the diet and in preferably no more than 0.5% by weight. The diet for animals may consist of normal foodstuffs to which the drug may be added or the drug may be included in a premix for admixture with the foodstuff.

A suitable method of administration of the drugto animals is to add it to the animals' drinking water. In this case a concentration of the drug in the drinking water of about 5-500 µg/ml, for example 5-200 µg/ml, is suitable.

The present invention further provides a method for treating the human, or non-human, animal which method comprises administering a compound of formula (I) as hereinbefore defined, to a human or non-human in need of such therapy.

Alternatively, a pharmaceutical composition as hereinbefore described may be employed in the treatment.

In particular aspects of the treatment there are provided methods for treating bacterial and/or mycoplasmal infections of human or non-human animals, especially venereal disease, respiratory infections, such as bacterial bronchitis, bacterial meningitis, non-specific urethritis and pneumonia in humans, respiratory infections, mastitis, swine dysentery and pneumonia in animals.

The following Examples illustrate the invention, but are not intended to limit the scope in any way.

In the Examples, the following abbreviation(s) are employed:

Tetrahydrofuran - THF

EXAMPLE 1

Preparation of Ethyl 4-[5S-(2S,3S-epoxy-5S-hydroxy-4S-methylhexyl)-3R,4R-dihydroxytetrahydropyran-2S-yl]-2,3-dimethylbut-2(E)-enoate (Ethyl 2-methylmonate A)

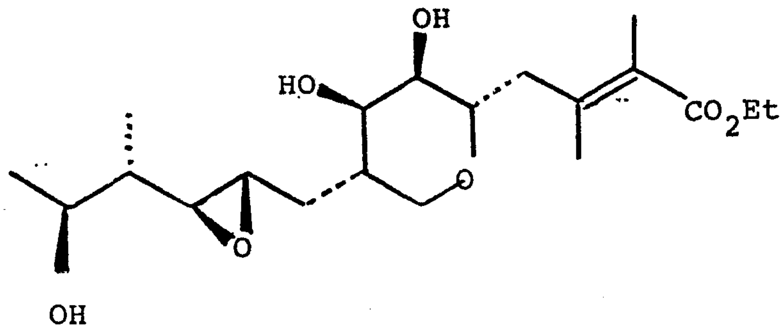

To a solution of ethyl monate (3.72 g, 10.0 mmol) and triethylamine (4.35 ml, 31.0 mmol) in dry THF (50 ml) was added trimethylsilyl chloride (3.95 ml, 31.0 mmol) and a catalytic amount of 4-(N,N-dimethylamino)-pyridine. After stirring at room temperature for 2 h, the triethylamine hydrochloride was filtered off and the solution concentrated under reduced pressure. The resultant "protected ester" obtained as an oil was dissolved in dry THF (20 ml) and refiltered ready for the next stage of the reaction.

A solution of lithium diisopropylamide (LDA) was prepared by reaction of butyl lithium (7.5 ml of a 1.6M solution) with diisopropylamine (1.70 ml, 12.0 mmol) in dry THF (20 ml) at -78°C for 15 mins.

The "protected ester", vide supra, was added dropwise to the LDA solution and stirred for 1 h at -78°C. Iodomethane (0.8 ml, 11.0 mmol) was added and the solution was allowed to warm to 0°C then stirred for a further 30 mins at this temperature before it was quenched with ammonium chloride solution, extracted with ethyl acetate (3 x 100 ml) the ethyl acetate layers being collected, combined and dried over magnesium sulphate. The solvent was removed under

reduced pressure and the residue was dissolved in *tert*-amyl alcohol (20 ml) and slowly added to a solution of sodium *tert*-amylate in *tert*-amyl alcohol (20 ml) [prepared from *tert*-amyl and sodium hydride, (480 mg, 10.0 mmol)] at 0°C. After stirring for 3 h at room temperature, the reaction was quenched with ammonium chloride. The reaction mixture was extracted with ethyl acetate (3 x 100 ml), the ethyl acetate layers being collected, combined and dried over magnesium sulphate before removal of solvent under reduced pressure to give an oil (5.31 g) which was chromatographed on silica using 5% ether in hexane as eluant to give starting material (2.08 g) and protected ethyl 2-methyl monate A (1.04 g). The latter was dissolved in THF/water (4:1, 100 ml) and treated with a few drops of hydrochloric acid for 5 minutes, then finally quenched with sodium bicarbonate.
The mixture was extracted with ethyl acetate, the ethyl acetate layer separated and dried over magnesium sulphate and the solvent removed under reduced pressure to give ethyl 2-methyl monate A (650 mg), which was further purified by column chromatography (silica, 0 - 5% methanol/dichloromethane) to yield pure ethyl 2-methyl monate A (190 mg, 0.49 mmol, 4.9%), which was crystallised from benzene to give fine white needles (mp 89-90°C).

i.r. spectrum

$\nu_{max}$ (liquid film) 3600 - 3200, 2970, 2930, 1700, 1635, 1450, 1370, 1280, 1215, 1100, 1050, 905 $cm^{-1}$;

U.V. spectrum

$\lambda_{max}$ 225 nm, $\epsilon_m$= 9,390);

$^{1}H$ nmr

$\delta_H$ ($CDCl_3$) 0.94 (3H, d, J = 7Hz, $CH_3$-17), 1.22 (3H, d, J = 7Hz, $CH_3$-14), 1.30 (3H, t, J = 7Hz, $OCH_2C\underline{H}_3$), 1.35 (1H, m, CH-12), 1.73 (2H, m, $CH_2$-9), 1.89 (3H, m, $CH_3$-2), 2.02 (4H, m, $CH_3$-15 +CH-8), 2.32 (1H, dd, J = 9, 15Hz, $CH_2$-4), 2.58 (1H, dd, J = 4, 15Hz, $CH_2$-4), 2.72 (1H, dd, J = 7, 1Hz, CH-11), 2.82 (1H, dt, J = 1, 7Hz, CH-10), 2.95 (1H, d, J = 8Hz), 3.11 (1H, s), 3.4 - 4.0 (6H, m), 4.19 (2H, q, J = 7Hz, $OC\underline{H}_2CH_3$);

$^{13}C$ nmr

$\delta_C$ ($CDCl_3$) 12.6 (C17), 14.3 ($OCH_2\underline{C}H_3$), 15.8 (C15), 20.8 (C14), 21.4 (C-2, $CH_3$), 31.8 (C9), 38.0 (C4), 39.6 (C8), 42.8 (C12), 55.7 (C10), 60.2 ($O\underline{C}H_2CH_3$), 61.3 (C11), 65.5 (C16), 69.6 (C6), 70.5 (C7), 71.2 (C13), 76.1 (C5), 124.8 (C3), 142.9 (C2), 170.3 (C1);

mass spectrum

m/e (relative intensity) 386 ($M^+$, 3%) 227 (69), 141 (56), 125 (66), 97 (75), 95 (63), 69 (100), 55 (64), 43 (96). ($M^+$ 386.2294, $C_{20}H_{34}O_7$ requires 386.2360).

EXAMPLE 2

Preparation of Methyl 4-[5S-(2S,3S-epoxy-5S-hydroxy-4S-methylhexyl)-3R,4R-dihydroxytetrahydropyran-2S-yl]-2,3-dimethylbut-2(E)-enoate (Methyl 2-methylmonate A)

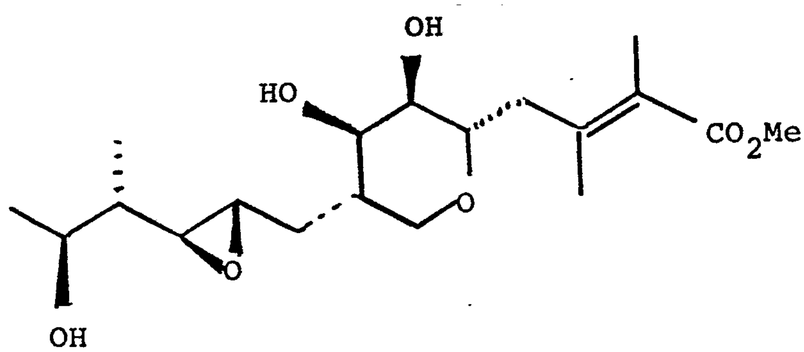

(a) Methyl 3-[(5-(2,3-epoxy-5-hydroxy-4-methylhexyl)-3,4-dihydroxytetrahydropyran-2-yl)methyl]-2-methyl-but-3-enoate

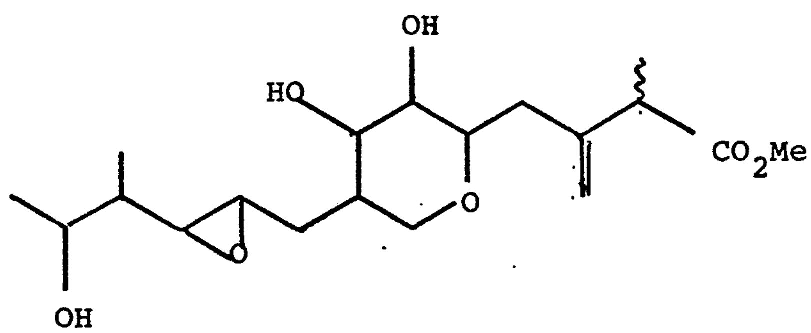

To a solution of methyl monate (716 mg, 2.0 mmol) and triethylamine (0.87 ml, 6.2 mmol) in dry THF (20 ml) was added trimethylsilyl chloride (0.79 ml, 6.2 mmol) and a catalytic amount of 4-(N,N-dimethylamino)-pyridine. After stirring for 2 h at room temperature the triethylamine hydrochloride was filtered off and the solution concentrated under reduced pressure. The resultant "protected ester", obtained as an oil was dissolved in dry THF (15 ml) and refiltered ready for the next stage of the reaction.

A solution of lithium diisopropylamide was prepared by the reaction of butyl lithium (1.88 ml of a 1.6M solution) with diisopropylamine (0.42 ml) in THF (10 ml) for 15 mins at -78°C. To this solution, still at -78°C, was added the tris(trimethylsilyl)-protected ester from above; stirring was continued for 1 h at -78°C and then iodomethane (0.63 ml, 10 mmol) was added. The cooling bath was allowed to warm to room temperature then the solution stirred for a further 30 mins and quenched with ammonium chloride solution. The mixture was extracted with ethyl acetate (3 x 50 ml), the ethyl acetate layers being collected, combined and dried (magnesium sulphate) before removal of solvent to give the crude, protected product, half of which was kept and used in parts (b) and (c) below; the other half was dissolved in THF/water (4:1, 100 ml) and treated with a few drops of hydrochloric acid for 5 mins then quenched with sodium bicarbonate. The resultant mixture was extracted with ethyl acetate, the ethyl acetate layer being collected and dried (magnesium sulphate). The solvent was removed under reduced pressure to give methyl 3-[(5-(2,3-epoxy-5-hydroxy-4-methylhexyl)-3,4-dihydroxytetrahydropyran-2-yl)methyl]-2-methyl-but-3-enoate as an oil (60 mg, 0.43 mmol, 43%).

<u>i.r. spectrum</u>

$\nu_{max}$ (liquid film) 3600 - 3200, 2960, 2920, 1725, 1640, 1450, 1255, 1120, 1070, 875 $cm^{-1}$;

<u>$^1H$ nmr</u>

$\delta_H$ ($CDCl_3$) 0.92 (3H, d, J = 7Hz, $CH_3$-17), 1.22 (3H, d, J = 7Hz, $CH_3$-14), 1.30 (3H, 2xd, J = 7Hz, $CH_3$-C2), 1.36 (1H, m, CH-12), 1.72 (2H, m, $CH_2$-9), 2.00 (1H, bm, CH-8), 2.24 (1H, m, CH-4), 2.55 (1H, m, CH-4), 2.74 (1H, dd, J = 7, 1Hz, CH-11), 2.82 (1H, dt, J = 1, 7Hz, CH-10), 3.25 (1H, q, J = 7Hz, CH-2), 3.4-3.95 (12H, m + s), 5.05 (2H, m);

<u>$^{13}C$ nmr</u>

$\delta_C$ ($CDCl_3$), 12.6 (C17), 16.2, 16.5 (Me, C2), 20.7 (C14), 31.7 (C9), 37.0, 37.1 (C4), 39.5 (C8), 42.8 (C12), 45.3, 45.9 (C2), 51.9 ($OCH_3$), 55.7 (C10), 61.2 (C11), 65.4 (C16), 68.9, 69.1 (C6), 70.4 (C7), 71.1 (C13), 75.2, 75.8 (C5), 113.4, 113.8 (C15), 144.7, 145.1 (C3), 175.3 (C1);

<u>mass spectrum</u> - (chemical ionisation using ammonia): <u>m/e</u> (relative intensity), 390 ($MNH_4^+$, 36%), 373 ($MH^+$, 100), 355 (72), 337 (50).

(b) <u>Methyl 4-[5S-(2S,3S-epoxy-5S-hydroxy-4S-methylhexyl)-3R,4R-dihydroxytetrahydropyran-2S-yl]-2,3-dimethylbut-2(E)-enoate</u>

The crude deconjugated tris(trimethylsilyl) protected ester (602 mg, 1.02 mmol, from above, prior to acid treatment) was dissolved in THF (5 ml), cooled to about -20°C to -30°C and treated with a solution of potassium <u>tert</u>-butoxide (112 mg, 1.0 mmol) in dry THF (10 ml). The mixture was stirred at that temperature for 30 mins then quenched with aqueous ammonium chloride. The resultant solution was allowed to warm to room temperature, and was extracted with ethyl acetate (2 x 50 ml). The organic layers were separated, combined and dried (magnesium sulphate). Removal of the solvent under reduced pressure gave the

crude product as an oil which was dissolved in THF/water (4:1, 10 mg/ml) and treated with concentrated hydrochloric acid (1 drop/5 ml) for 5 mins then quenched with sodium bicarbonate solution. The resultant mixture was extracted with ethyl acetate. The ethyl acetate layer was separated and dried (magnesium sulphate), and the solvent removed under reduced pressure to give the crude product (420 mg) as a 71:29 mixture of E and Z isomers. Purification of the major isomer by chromatography gave methyl 2-methyl monate A (118 mg, 0.32 mmol, 31%, total yield 247 mg, 0.66 mmol, 65%).

<u>i.r. spectrum</u>

$\nu_{max}$ (film) 3600-3200, 2980, 2930, 1710, 1640, 1450, 1380, 1290, 1220, 1110, 1060, 930, 900 $cm^{-1}$;

<u>U.V. spectrum</u>

$\lambda_{max}$ (EtOH) 226 nm ($\varepsilon_m$ 6880);

<u>$^1H$ nmr</u>

$\delta_H$ ($CDCl_3$) 0.94 (3H, d, J = 7Hz, $CH_3$-17), 1.21 (3H, d, J = 7Hz), 1.34 (1H, q, J = 7Hz, CH-12), 1.73 (2H, m, CH-9), 1.89 (3H, d, J = 2Hz, <u>$CH_3$</u>-C2), 2.00 (1H, m, CH-8), 2.02 (3H, d, J = 2Hz, $CH_3$-15), 2.32 (1H, dd, J = 15, 9Hz, $CH_2$-4), 2.58 (1H, dd, J = 15, 3Hz, $CH_2$-4), 2.72 (1H, dd, J = 8, 2Hz, CH-11), 2.82 (1H, dt, J = 2, 5Hz, CH-10), 3.4-4.0 (7H, m + 3H, s, $CO_2CH_3$);

<u>$^{13}C$ nmr</u>
$\delta_C$ ($CDCl_3$) 12.6 (C17), 15.8 ($CH_3$-C2), 20.8 (C14), 21.5 (C15), 31.8 (C9), 38.1 (C4), 39.6 (C8), 42.8 (C12), 51.3 ($CO_2\underline{C}H_3$), 55.7 (C10), 61.3 (C11), 65.5 (C16), 69.6 (C6), 70.5 (C7), 71.1 (C13), 76.2 (C5), 124.2 (C2), 143.7 (C3), 170.6 (C1);

<u>mass spectrum</u>
<u>m/e</u> (relative intensity) 372 (2%, $M^+$) 227 (65), 209 (23), 141 (47), 125 (57), 111 (53), 97 (62), 95 (57), 69 (95), 55 (62), 43 (100) (Found: 372.2124. $C_{19}H_{32}O_7$ requires 372.2102).

(c) <u>Methyl 4-[(5S-(2S,3S-epoxy-5S-hydroxy-4S-methylhexyl)-3R,4R-dihydroxytetrahydropyran-2S-yl]-2,3-dimethyl-2(Z)-enoate</u> (Methyl 2-methylisomonate A)

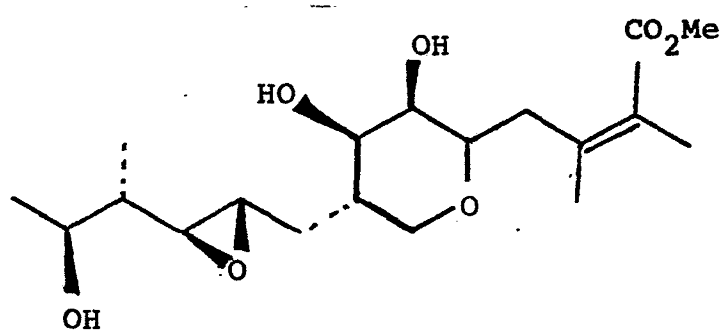

The crude deconjugated tris(trimethylsilyl) protected ester (1.08 g, 1.79 mmol, from above, prior to acid treatment) and 1,8-diazabicyclo[5.4.0]undec-7-ene (DBU, 2.7 ml, 18 mmol) in dry THF (20 ml) were heated under reflux for 24 h, cooled then quenched with excess aqueous ammonium chloride. The resultant solution was extracted with ethyl acetate (2 x 50 ml) and the organic layers separated, combined and dried (magnesium sulphate). The solvent was removed under reduced pressure to give the crude product as an oil which was dissolved in THF/water (4:1, 10 mg/ml) and

treated with concentrated hydrochloric acid (1 drop/5 ml) for 5 mins then quenched with sodium bicarbonate solution. The mixture was extracted with ethyl acetate, the ethyl acetate layer was separated and dried (magnesium sulphate) and the solvent was removed under reduced pressure to give the crude product (658 mg) as a 21:79 mixture of the E and Z isomers. Purification of the major isomer by chromatography gave methyl 2-methyl isomonate A (312 mg, 0.84 mmol, 47%) which slowly crystallised from ether (mp 83.5-84°C).

i.r. spectrum

$\nu_{max}$ (film) 3600-3200, 2970, 2920, 1700, 1630, 1435, 1380, 1280, 1195, 1100, 1050, 900 $cm^{-1}$;

$^{1}H$ nmr

$\delta_H$ ($CDCl_3$) 0.95 (3H, d, J = 7Hz, $CH_3$-17), 1.21 (3H, d, J = 7Hz, $CH_3$-14), 1.30 (1H, q, J = 7Hz, CH-12), 1.55-1.85 (2H, m, $CH_2$-9), 1.89 (3H, bs, Me-C2), 1.92 (3H, s, $CH_3$-15), 2.00 (1H, m, CH-8), 2.63, 2.75 (2H, m, $CH_2$-4), 2.67 (1H, dd, J = 8, 2Hz, CH-11), 2.79 (1H, dt, J = 2, 5Hz, CH-10), 3.4-4.0 (5H, m), 3.73 (3H, s, $CO_2CH_3$);

$^{13}C$ nmr

$\delta_C$ ($CDCl_3$) 12.5 (C17), 15.8 ($CH_3$-C2), 20.7 (C14), 21.5 (C15), 32.0 (C9), 38.6 (C4), 39.2 (C8), 42.9 (C12), 51.8 ($OCH_3$), 55.8 (C10), 61.2 (C11), 65.6 (C16), 68.7 (C6), 70.4 (C7), 71.0 (C13), 76.6 (C5), 124.2 (C2), 146.5 (C3), 171.0 (C1);

mass spectrum

m/e (relative intensity) 372 (0.5%, $M^+$) 227 (74), 125 (96), 111 (50), 97 (79), 96 (54), 95 (54), 69 (94), 55 (62), 43 (100) (Found: 372.2163. $C_{19}H_{32}O_7$ requires 372.2178).

EXAMPLE 3

m-Nitrobenzyl 4-[5S-(2S,3S-epoxy-5S-hydroxy-4S-methylhexyl)-3R,4R-dihydroxytetrahydropyran-2S-yl]-2,3-dimethylbut-2(E)-enoate (m-Nitrobenzyl 2-methylmonate A)

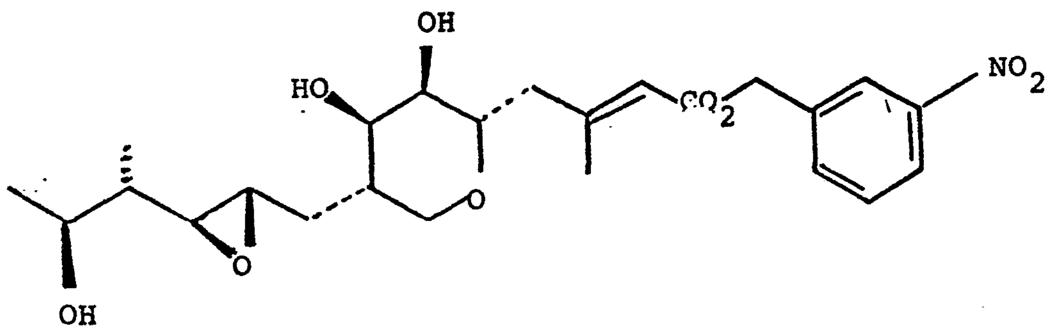

(a) m-Nitrobenzyl 3-[(5-(2,3-epoxy-5-hydroxy-4-methylhexyl)-3,4-dihydroxytetrahydropyran-2-yl)methyl] -2-methylbut-3-enoate

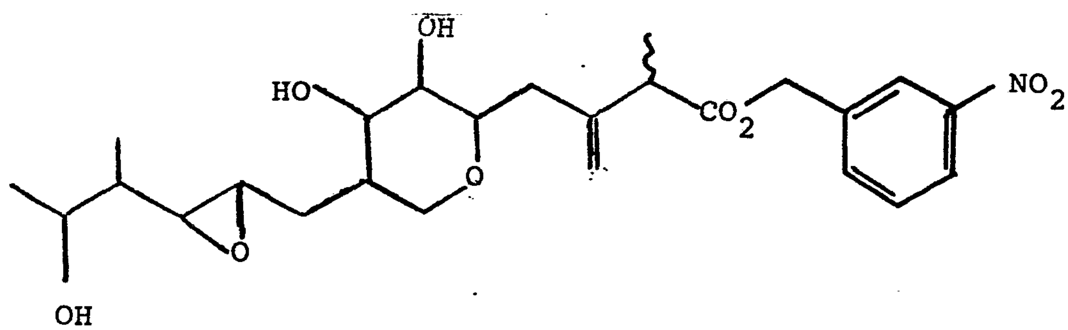

To a solution of monic acid A (1.72 g, 5.0 mmol) and triethylamine (3.14 ml, 22.5 mmol) in dry THF was added trimethylsilyl chloride (2.85 ml, 22.5 mmol) and a catalytic amount of 4-(N,N-dimethylamino)pyridine. After stirring at room temperature for 2 h the triethylamine hydrochloride was filtered off and the solution and the solution concentrated under reduced pressure to give the "protected acid" as an oil which was dissolved in dry THF and refiltered ready for the next stage of the reaction.

A solution of lithium diisopropylamide (LDA) was prepared by reaction of butyl lithium (4.84 ml of a 1.55M solution) with diisopropylamine (1.05 ml, 7.50 mmol) in dry THF (10 ml) at -78°C for 15 min. The "protected acid", vide supra, was added dropwise to the LDA solution and the mixture was stirred for 1 h at -78°C. Methyl iodide (0.4 ml, 5.5 mmol) was added and the cooling bath was allowed to warm to room temperature. The solution was then stirred overnight, quenched with ammonium chloride solution, extracted with ethyl acetate (3 x 50 ml) and the ethyl acetate layers were separated, combined and dried (magnesium sulphate). The solvent was removed under reduced pressure to give the crude "deconjugated acid".

The sodium salt of this was prepared by reacting the "deconjugated acid" in methanol/water with sodium bicarbonate (460 mg, 5.5 mmol). The mixture was treated with m-nitrobenzyl choride (1.29 g, 7.5 mmol) in N,N-dimethylformamide (10 ml) at room temperature for 24 h to give the crude product. This material was deprotected by treatment with acid and then purified by column chromatography on silica gel using dichloromethane/methanol (0-3%) as eluant to yield m-Nitrobenzyl 3-[(5-(2,3-epoxy-5-hydroxy-4-methyl-hexyl)-3,4-dihydroxytetrahydropyran-2-yl) methyl]-2-methylbut-3-enoate the "deconjugated ester" as a mixture of diastereomers (1.00 g, 2.03 mmol, 41%).

i.r. spectrum

$\nu_{max}$ (film) 3600-3200, 2970, 2930, 1740, 1645, 1530, 1350, 1215, 1090, 1040, 900, 800 $cm^{-1}$;

U.V. spectrum

$\lambda_{max}$ (EtOH) 210 nm ($\epsilon_m$ 11,900);

$^1H$ nmr

$\delta_H$ ($CDCl_3$) 0.93 (3H, d, J = 7Hz, $CH_3$-17), 1.21 (3H, d, J = 7Hz, $CH_3$-14), 1.34 (4H, m + 2xd, CH-12 + $CH_3$-C2), 1.70 (2H, t, J = 7Hz, $CH_2$-9), 1.99 (1H, bm, CH-8), 2.27 (1H, m, $CH_2$-4), 2.55 (1H, bd, J = 12Hz, $CH_2$-4), 2.72 (1H, m, CH-11), 2.80 (2H, m), 3.33 (1H, q, J = 7Hz, CH-2), 3.40-4.00 (6H, m), 5.09 (2H, m, $CH_2$-15), 5.22 (2H, ABq, $CH_2$-Ar), 7.55 (1H, t, J = 8Hz, $C_5$-H, Ar), 7.68 (1H, d, J = 8Hz, $C_6$-H, Ar), 8.18 (1H, d, J = 8Hz, $C_4$-H, Ar), 8.20 (1H, s, $C_2$-H, Ar);

$^{13}C$ nmr

$\delta_C$ ($CDCl_3$) 12.6 (C17), 16.3, 16.5 ($CH_3$-C2), 20.8 (C14), 31.8 (C9), 37.3 (C4), 39.6 (C8), 42.8 (C12), 45.4, 45.8 (C2), 55.7 (C10), 61.2 (C11), 64.9 ($CH_2$), 65.4 (C16), 69.1 (C6), 70.5 (C7), 71.2 (C13), 75.4, 76.0 (C5), 113.7, 114.1 (C15), 122.6, 123.0 (C2', C4', Ar), 129.6 (C5', Ar), 133.7 (C6', Ar), 138.5 (C1', Ar), 144.6, 145.1 (C3), 148.6 (C3', Ar), 174.2 (C1);

mass spectrum (chemical ionisation with ammonia): m/e (relative intensity) 512 (32%, $MNH_4^+$), 494 (47, $MH^+$), 464 (100), 359 (50), 341 (65), 227 (58), 106 (33).

(b) m-Nitrobenzyl 4-[5S-(2S,3S-epoxy-5S-hydroxy-4S-methylhexyl)-3R,4R-dihydroxytetrahydropyran-2S-yl]-2,3-dimethylbut-2-enoate

The deconjugated ester from part (a) (2.12 g, 4.3 mmol) was protected in the manner described in part (a) using triethylamine (1.85 ml, 13.3 mmol) and trimethylsilyl chloride (1.69 ml, 13.3 mmol) and a catalytic amount of 4-(N,N-dimethylamino)pyridine. This crude material was then treated with DBU (6.4 ml, 43 mmol) in dry THF (20 ml) as described in Example 2(c). The protected ester was treated with acid as in Example 1 to yield the crude product (1.81 g) as

a mixture of E and Z isomers in the ratio of ca 55:45. Column chromatography allowed isolation of both isomers:

m-nitrobenzyl 2-methylisomonate (the Z isomer) (mp 97-98°C) (from diethyl-ether).

i.r spectrum

$\nu_{max}$ (film) 3600-3200, 2970, 2930, 1710, 1640, 1530, 1450, 1350, 1270, 1180, 1090, 905, 805 $cm^{-1}$;

U.V. spectrum

$\lambda_{max}$ (EtOH) 204 nm ($\varepsilon_m$ 28,400);

$^1H$ nmr

$\delta_H$ ($CDCl_3$) 0.92 (3H, d, J = 7Hz, $CH_3$-17), 1.22 (3H, d, J = 7Hz, $CH_3$-14), 1.32 (1H, q, J = 7Hz, CH-12), 1.55-1.80 (2H, m, $CH_2$-9), 1.95 (6H, s, $CH_3$-15 + $CH_3$-C2), 2.00 (1H, m, CH-8), 2.50-2.85 (4H, m), 3.40-4.00 (6H, m), 5.25 (2H, s, $CH_2$-Ar), 7.56 (1H, t, J = 8Hz, $C_5$-H, Ar), 7.70 (1H, d, J = 8Hz, $C_6$-H, Ar), 8.19 (1H, d, J = 8Hz, $C_4$-H, Ar), 8.24 (1H, s, $C_2$-H, Ar);

$^{13}C$ nmr

$\delta_C$ ($CDCl_3$) 12.5 (C17), 15.8 ($CH_3$-C2), 20.6 (C14), 21.7 (C15), 31.8 (C9), 38.3 (C4), 39.1 (C8), 42.7 (C12), 55.6 (C10), 61.1 (C11), 64.9 ($CH_2$), 65.5 (C16), 68.8 (C6), 70.3 (C7), 70.9 (C13), 76.4 (C5), 122.7, 123.0 (C2', C4', Ar), 123.5 (C2), 129.7 (C5', Ar), 133.8 (C6', Ar), 138.2 (C1', Ar), 148.0 (C3), 148.4 (C3', Ar), 169.7 (C1);

mass spectrum

m/e (relative intensity) 493 (0.5%, $M^+$) 227 (55), 136 (70), 125 (88), 97 (55), 69 (76), 55 (64), 43 (100);

m-nitrobenzyl 2-methylmonate (the E isomer), mp 96-103°C (ethanol):

i.r. spectrum

$\nu_{max}$ 3600-3200, 2970, 2930, 1710, 1630, 1530, 1450, 1350, 1205, 1095, 910, 805 $cm^{-1}$;

U.V. spectrum

$\lambda_{max}$ (EtOH) 217 nm $\epsilon_m$ 14,920);

$^1H$ nmr

$\delta_H$ ($CDCl_3$) 0.94 (3H, d, J = 7Hz, $CH_3$-14), 1.33 (1H, m, CH-12), 1.74 (2H, m, $CH_2$-9), 1.97 (3H, s, $CH_3$-C2), 2.00 (1H, m, CH-8), 2.09 (3H, s, $CH_3$-15), 2.36 (1H, dd, J = 14, 8Hz, $CH_2$-4), 2.61 (1H, dd, J = 14, 3Hz, $CH_2$-4), 2.72 (1H, dd, J = 8, 2Hz, CH-11), 2.80 (1H, dt, J = 2, 5Hz, CH-10)m 3.40-4.00 (6H, m), 5.27 (2H, s, $CH_2$-Ar), 7.55 (1H, t, J = 8Hz, $C_5$-H, Ar), 7.72 (1H, d, J = 8Hz, $C_6$-H, Ar), 8.20 (1H, d, J = 8Hz, $C_4$-H, Ar), 8.24 (1H, s, $C_2$-H, Ar);

$^{13}C$ nmr

$\delta_C$ ($CDCl_3$) 12.6 (C17), 15.7 ($CH_3$-C2), 20.8 (C14), 21.7 (C15), 31.7 (C9), 38.4 (C4), 39.6 (C8), 42.8 (C12), 55.6 (C10), 61.3 (C11), 64.5 ($CH_2$), 65.5 (C16), 69.4 (C6), 70.3 (C7), 71.2 (C13), 76.0 (C5), 122.7, 123.0 (C2', C4', Ar), 123.6 (C2), 129.6 (C5', Ar), 133.9 (C6', Ar), 138.6 (C1', Ar), 146.1 (C3), 148.4 (C3', Ar), 169.1 (C1);

m/e (relative intensity) 493 (0.5%, $M^+$), 227 (38), 136 (94), 125 (64), 90 (49), 71 (52), 69 (95), 55 (70), 43 (100).

Example 4

Sodium 4-[5S-(2S, 3S-epoxy-5S-hydroxy-4S-methylhexyl)-3R,4R-dihydroxytetrahydropyran-2S-yl]-2,3-dimethylbut-2(E)-enoate (Sodium 2-methylmonate A)

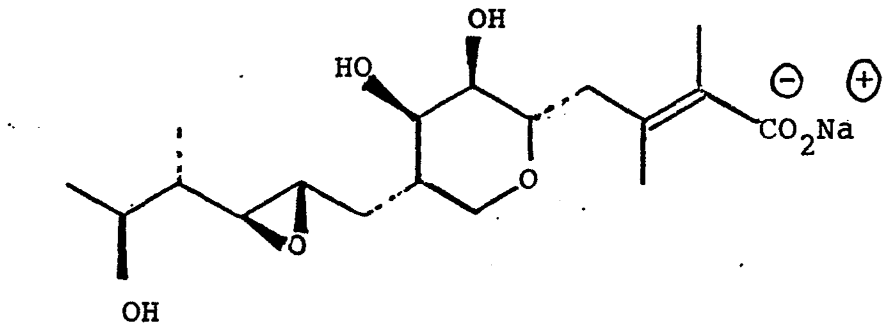

Ethyl 2-methylmonate (448 mgs, 1.16 mmol) was dissolved in trimethyl orthoformate (10 ml), a catalytic amount of p-toluene sulphonic acid added and the solution stirred for 30 min at room temperature. The reaction mixture was then poured into water, extracted with ethyl acetate (3 x 50 ml), the combined organic layers washed with sodium bicarbonate solution and brine, then dried (magnesium sulphate). The solvent was removed under reduced pressure to give an oil which was dissolved in methanol (10 ml) and treated with sodium hydroxide solution (10 ml, 1M) at 80°C for 2 h. The pH was then adjusted to pH 2 with dilute hydrochloric acid for 10 min, to pH 9.5 with dilute sodium hydroxide for 30 min, then to pH 7 and the methanol was removed under reduced pressure. Finally the pH was adjusted to pH 4 at which point the mixture was rapidly extacted with ethyl acetate (3 x 50 ml). The ethyl acetate layers were separated, combined and dried (magnesium sulphate) and the solvent removed under reduced pressure to give the crude acid as an oil which was dissolved in a water/methanol mixture (5 ml,

1:1) and sodium bicarbonate (97.5 mg, 1.16 mmol) was added. After 30 min at room temperature the solvent was removed under reduced pressure, the residue dissolved in methanol, filtered, and the solvent removed to yield sodium 2-methyl monate A (285 mg, 0.75 mmol, 65%);

<u>i.r. spectrum</u>
$\nu_{max}$ (film) 3700-3200, 2970, 2920, 1550, 1450, 1400, 1100, 1030 $cm^{-1}$;

<u>U.V. spectrum</u>
$\lambda_{max}$ (EtOH) 224 nm ($\epsilon_m$ 6,460);

<u>$^1H$ nmr</u>
$\delta_H$ ($CD_3OD$), 0.95 (3H, d, J = 7Hz, $CH_3$-17), 1.22 (3H, d, J = 7Hz, $CH_3$-14), 1.39 (1H, m, CH-12), 1.70 (2H, m, $CH_2$-9), 1.81 (3H, s, $CH_3$-C2), 1.85 (3H, s, $CH_3$-15), 1.95 (1H, m, CH-8), 2.12 (1H, dd, J = 14, 9Hz, $CH_2$-4), 2.49 (1H, dd, J = 9, 3Hz, $CH_2$-4), 2.70 (1H, dd, J = 10, 2Hz, CH-11), 2.82 (1H, dt, J = 2, 6Hz, CH-10), 3.4-4.0 (6H, m);

<u>$^{13}C$ nmr</u>
$\delta_C$ ($CD_3OD$) 12.3 (C17), 16.6 (C15), 20.5 (C14), 21.0 ($CH_3$-2), 33.0 (C9), 36.3 (C4), 41.1 (C8), 43.7 (C12), 56.9 (C10), 61.6 (C11), 66.2 (C16), 70.5 (C6), 70.9 (C7), 71.7 (C13), 77.7 (C5), 127.9 (C2), 133.8 (C3), 170.5 (C1).

EXAMPLE 5

m-Cyanobenzyl 4-[5S-(2S, 3S-epoxy-5S-hdroxy-4S-methylhexyl)-3R, 4R-dihydroxytetrahydropyran-2S-yl]-2,3-dimethylbut-2(E)-enoate (m-cyanobenzyl 2-methylmonate A)

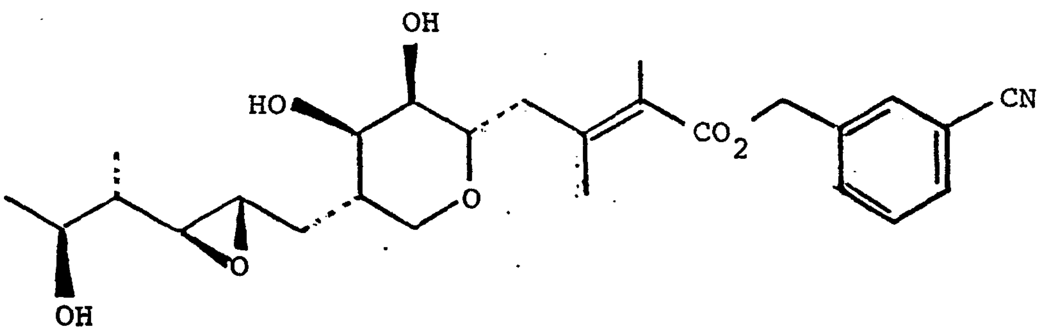

A solution of sodium 2-methylmonate A (110 mg, 0.29 mmol) and m-cyanobenzyl bromide (85 mg, 0.43 mmol) in N,N-dimethyl formamide (2 ml) stirred at room temperature for 15h. The solvent was removed under reduced pressure to give an oily residue which was dissolved in ethyl acetate, washed with sodium bicarbonate and brine then dried (magnesium sulphate). The solvent was removed under reduced pressure to give the crude product which was purified by column chromatography on silica gel (2 g) using methanol in dichloromethane (0-5%) as eluant to yield m-cyanobenzyl 2-methyl monate A (25.4 mg, 0.053 mmol, 18.5%).

i.r. spectrum

$\nu_{max}$ ($CHCl_3$) 3600-3200, 3000, 2960, 2230, 1705, 1375, 1190, 1090 $cm^{-1}$;

U.V. spectrum

$\lambda_{max}$ (EtOH) 227 nm ($\varepsilon_m$ 22,930);

$^1H$ nmr

$\delta_H$ ($CDCl_3$), 0.94 (3H, d, J = 7Hz, $CH_3$-17), 1.22 (3H, d, J = 7Hz, $CH_3$-14), 1.34 (1H, m, CH-12), 1.75 (2H, m, $CH_2$-9), 1.94 (3H, bs, $CH_3$-C2), 2.02 (1H, m, CH-8), 2.06 (3H, bs, $CH_3$-15), 2.37 (1H, dd, J = 14, 8Hz, $CH_2$-4), 2.60 (1H, dd, J = 14, 3Hz, $CH_2$-4), 2.72 (1H, dd, J = 10, 2Hz, CH-11), 2.82 (1H, dt, J = 2, 7Hz, CH-10), 3.4-4.0 (6H, m), 5.20 (2H, s, $CH_2$), 7.4-7.7 (4H, m, Ar);

$^{13}C$ nmr

$\delta_C$ ($CDCl_3$), 12.7 (C17), 15.8 ($CH_3$-C2), 20.9 (C14), 21.7 (C15), 31.7 (C9), 38.4 (C4), 39.7 (C8), 42.9 (C12), 55.6 (C10), 61.4 (C11), 64.6 ($CH_2$), 65.4 (C16), 69.6 (C6), 70.5 (C7), 71.4 (C13), 76.0 (C5), 112.7 (C3'), 118.5 (CN), 123.8 (C2), 129.4, 131.4, 131.7, 132.3 (C2', 4', 5', 6'), 138.2 (C1'), 145.8 (C3), 169.1 (C1);

mass spectrum

m/e (relative intensity) 473 ($M^+$, 1%), 227 (25), 116 (100), 97 (33), 69 (57), 57 (35), 55 (44), 43 (72), 41 (63). (Found 473.2440 $C_{26}H_{35}NO_7$ requires 473.2413).

Example 6

Methyl 9-[4-(5S-(2S,3S-epoxy-5S-hydroxy-4S-methylhexyl-3R,4R-dihydroxytetrahydropyran-2S-yl)-2,3-dimethylbut-2(E)-enoyloxy]nonanoate (Methyl 2-methylpseudomonate A)

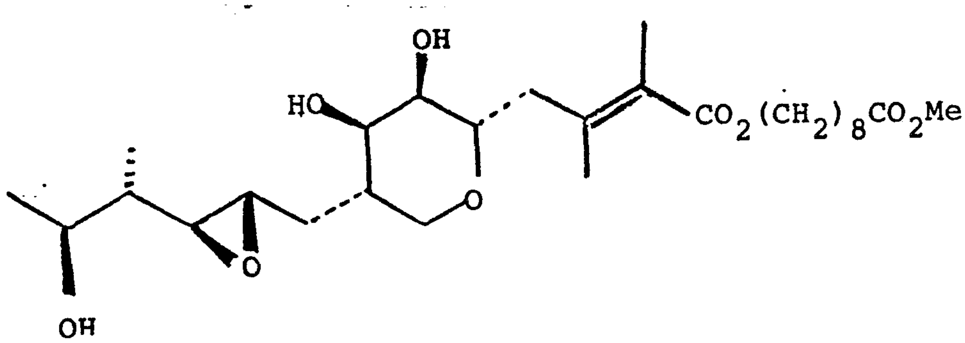

A solution of sodium 2-methylmonate A (174 mg, 0.46 mmol) produced as described in Example 4 part (a), methyl 9-chlorononanoate (189 mg, 0.92 mmol), sodium iodide (138 mg, 0.92 mmol) and hexamethyl-phosphoric triamide (2 drops) in N,N-dimethyl formamide (3 ml) was heated at 100°C for 3 h. Removal of the solvent under reduced pressure gave an oil which was dissolved in ethyl acetate, washed with sodium bicarbonate and brine then dried (magnesium sulphate). The solvent was removed under reduced pressure to give the crude product which was purified by column chromatography on silica (0-5% methanol in dichloromethane) to yield methyl 2-methylpseudomonate A (102 mg, 0.19 mmol, 42%).

i.r. spectrum

$\nu_{max}$ (film) 3600-3200, 2930, 2860, 1735, 1710, 1450, 1375, 1280, 1210, 1050 $cm^{-1}$;

U.V. spectrum

$\lambda_{max}$ (EtOH) 225 nm ($\varepsilon_m$ 10,050);

$^1H$ nmr

$\delta_H$ ($CDCl_3$), 0.93 (3H, d, J = 7Hz, $CH_3$-17), 1.21 (3H, d, J = 7Hz, $CH_3$-14), 1.30 (9H, bs), 1.62 (4H, m), 1.74 (2H, t, J = 6Hz, $CH_2$-9), 1.90 (3H, m, $CH_3$-C2), 2.02 (4H, m, $CH_3$-15 + CH-8), 2.32 (3H, m, $CH_2$-4 + $CH_2$-8'), 2.56 (1H, m, $CH_2$-4), 2.72 (1H, dd, J = 8, 2Hz, CH-11), 2.81 (1H, dt, J = 2, 6Hz, CH-10), 3.4-4.0 (9H, m + s), 4.12 (2H, t, J = 7Hz, $CH_2$-1');

$^{13}C$ nmr

$\delta_C$ ($CDCl_3$), 12.6 (C17), 15.8 ($CH_3$-2), 20.8 (C14), 21.5 (C15), 24.9 (C3'), 26.0 (C3'), 28.7 (C8'), 29.1 (C4', 5', 6'), 31.8 (C9), 34.1 (C2'), 38.1 (C4), 39.7 (C8), 42.8 (C12), 51.4 ($OCH_3$), 55.6 (C10), 61.3 (C11), 64.4 (C9'), 65.5 (C16), 69.6 (C6), 70.5 (C7), 71.1 (C13), 76.1 (C5), 124.7 (C2), 143.3 (C3), 170.2 (C1), 174.3 (C1');

mass spectrum

m/e (relative intensity) 528 ($M^+$, 1.5%), 227 (48), 125 (57), 97 (52), 96 (100), 95 (35), 69 (70), 55 (54), 45 (47) (Found 528.3282 $C_{28}H_{48}O_9$ requires 528.3297).

Example 7

Sodium 9-[4-(5S-(2S,3S-epoxy-5S-hydroxy-4S-methylhexyl)-3R,4R-dihydroxytetrahydropyran-2S-yl)-2,3-dimethylbut-2(E)-enoyloxy]nonanoate (sodium 2-methylpseudomonate A)

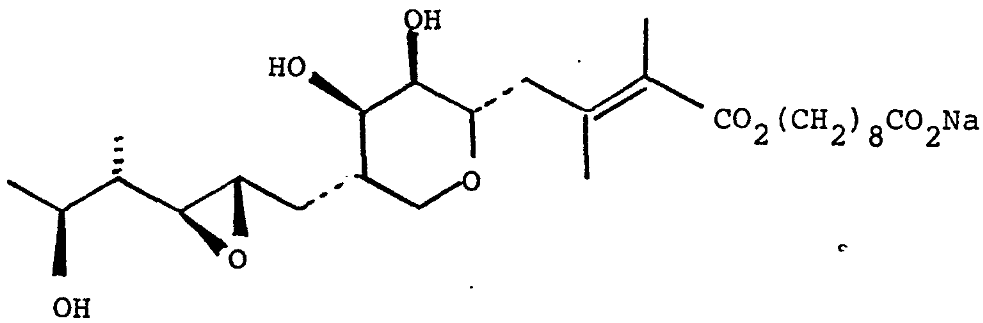

To a solution of methyl 2-methylpseudomonate A (34 mg, 64 µmol) produced as described in Example 5, in N,N-dimethyl formamide (1.1 ml) and pH 7 buffer (0.05M, 15 ml) was added bakers yeast (1.25 g), and the resultant suspension was stirred vigorously overnight. The yeast was removed by filtration, then the filtrate was evaporated to dryness. The residue was dissolved in water (ca 10 ml), acidified to pH 4 and rapidly extracted with ethyl acetate (2 x 50 ml). The ethyl acetate layers were separated, combined and then dried (magnesium sulphate). The solvent was removed under reduced pressure to give an oil which was dissolved in methanol/water (1:1, 2 ml) and treated with sodium bicarbonate (10.8 mg, 128 µmol). The solvent was removed to yield the sodium salt which was dissolved in methanol, filtered and evaporated to yield sodium 2-methylpseudomonate A as an oil (24.7 mg, 46.1 µmol, 72%).

i.r. spectrum

$\nu_{max}$ (film) 3600-3200, 2920, 1710, 1640, 1560, 1440, 1410, 1100 $cm^{-1}$;

U.V. spectrum

$\lambda_{max}$ (EtOH) 223.5 nm $\varepsilon_m$ 9, 080).

Example 8

Ethyl 4-[5S-(2S,3S-epoxy-5S-hydroxy-4S-methylhexyl) -3R,4R-dihydroxytetrahydropyran-2S-yl]-2,3-dimethylbut-2-enoate; ethyl 2-methylmonate A

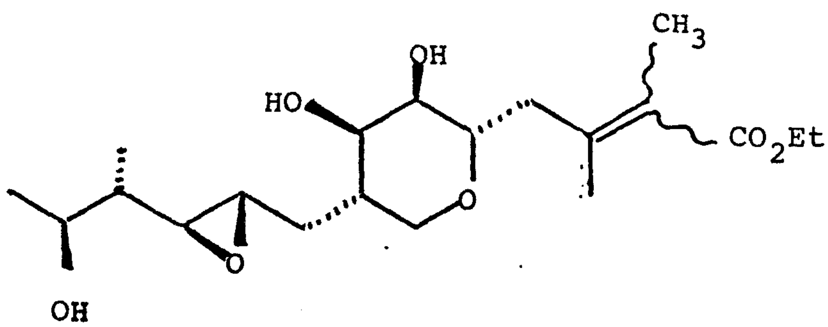

To a solution of 3R,4R-dihydroxy-5S-(2S,3S-epoxy-5S-hydroxy-4S-methylhexyl)tetrahydropyran-2S-yl acetone (604 mg, 2.0 mmol) in dry THF (20 ml) was added triethylamine (0.87 ml, 6.20 mmol), trimethylsilyl chloride (0.78 ml, 6.20 mmol) and a catalytic amount of 4-(N,N-dimethylamino)pyridine. After stirring at room temperature for 2h the triethylamine hydrochloride was filtered off and the solution concentrated under reduced pressure to afford an oil. The resultant "tris(trimethylsilyl) protected ketone" was taken up in anhydrous ether, filtered, solvent removed under reduced pressure then the oil taken up in dry THF ready for the next stage of the reaction.

To a solution of lithium diisopropylamide (from diisopropylamine, 0.42 ml, 3.00 mmol and n-butyl lithium, 1.9 ml, of a 1.6M solution) in THF (10 ml) at -78°C was added ethyl 2-(triethylsilyl)propionate (520 mg, 3.00 mmol) in THF (5 ml). After stirring the mixture for 2h at -78°C the tris(trimethylsilyl) protected ketone, vide supra, was added and the reaction mixture stirred for 30 minutes at -78°C then the reaction mixture stirred for 30 minutes a room temperature. The mixture was quenched with ammonium chloride then extracted with etyl acetate and dried (magnesium sulphate). Solvent removal under reduced

pressure gave an oil which was taken up in THF/water (100 ml, 4:1) and treated with acid (10 drops, concentrated hydrochloric acid) for 5 minutes. After this time the mixture was quenched with sodium bicarbonate solution and extracted with ethyl acetate. Drying (magnesium sulphate) and solvent removal under reduced pressure gave the crude produce (765 mg) as a mixture of E and Z-isomers. Column chromatography on silica (0-5% methanol in dichloromethane) gave ethyl 2-methylmonate A (149 mg, 0.38 mmol. 19%) identical to the material isolated in Example 1, and ethyl 2-methylisomonate A (455 mgs, 1.15 mmol, 58%):

i.r. spectrum

$\nu_{max}$ (film) 3600-3200, 2980, 2920, 1690, 1625, 1450, 1380, 1275, 1190, 1090, 900, 750 $cm^{-1}$;

U.V. spectrum

$\lambda_{max}$ (EtOH) 227 nm ($\varepsilon_m$ 7,440);

$^1H$ nmr

$\delta_H$ ($CDCl_3$) 0.95 (3H, d, J=7Hz, $CH_3$-17), 1.25 (7H, m, $CH_3$-14, $OCH_2C\underline{H}_3$ + CH-12), 1.55 (2H, m, $CH_2$-9), 1.80 (6H, s, $CH_3$-15 + $CH_3$-C2), 1.95 (1H, m, CH-8), 2.50-2.70 (4H, m), 3.2-3.90 (6H, m), 4.15 (2H, q, J=7Hz, $OCH_2C\underline{H}_3$).

Example 9

Photochemical isomerisation of ethyl 2-methylisomonate A

A solution of ethyl 2-methylisomonate A (90 mg, 0.23 mmol) in benzene (150 ml) was purged with argon and irradiated with ultra violet light (254 nm, low pressure mercury lamp, 6 watts) for 18h. Solvent removal under reduced pressure afforded the crude mixture of isomers (92 mg) as a pale yellow oil which were separated by column chromatography as described in Example 8. Hplc showed a mixture of ethyl 2-methylisomonate and ethyl 2-methylmonate in the ratio 51:49.

0090603

Example 10

3-[(5-(2,3-epoxy-5-hydroxy-4-methylhexyl)-3,4-dihydroxy -tetrahydropyran-2-yl)methyl]-2-methylbut-3-enoic acid

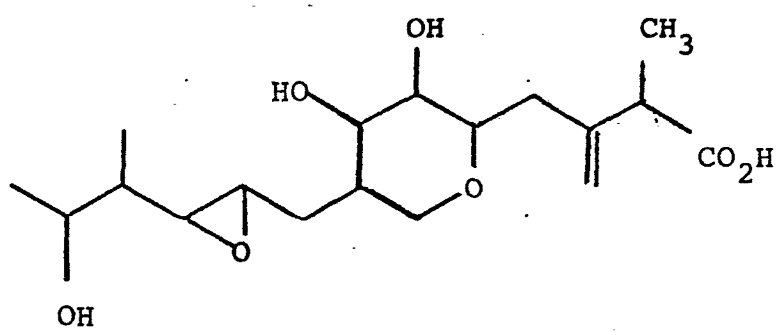

To a solution of monic acid (1.72 g, 5.00 mmol) and triethylamine (3.14 ml, 22.5 mmol) in dry THF was added trimethylsilyl chloride (2.85 ml, 22.5 mmol) and a catalytic amount of 4-(N,N-dimethylamino)pyridine. After stirring at room temperature for 2 h the triethylamine hydrochloride was filtered off and the solution concentrated under reduced pressure to an oil. The resultant "protected trimethysilyl ester" was taken up in dry THF and refiltered ready for the next stage of the reaction.

A solution of lithium diisopropylamide (LDA) was prepared by reaction of butyl lithium (4.84 ml of a 1.55 M solution) with diisopropylamine (1.05 ml, 7.50 mmol) in dry THF (10 ml) at -78°C for 15 min. The protected trimethylsilyl ester, vide supra, was added dropwise to the LDA solution and stirred for 1 h at -78°C before adding methyl iodide (0.4 ml, 5.50 mmol). The solution was allowed to warm to room temperature in the cooling bath, then stirred overnight, quenched with ammonium chloride solution, extracted with ethyl acetate (3 x 50 ml) and dried (magnesium sulphate). Solvent removal under reduced pressure gave crude 3-[(5-(2,3-epoxy-5-hydroxy-4-methylhexyl)-3, 4-dihydroxytetrahydropyran-2-yl)methyl]-2-methylbut-3-enoic acid (3.54 g).

Part of this material (708 mg) was taken up in THF/water (4:1, 10 mg/ml) and treated with concentrated hydrochloric acid (1 drop/5 ml) for 7 min. The pH was reduced to 3.5 (dilute hydrochloric acid) and the solution extracted rapidly with ethyl acetate (3 x 50 ml). Drying (magnesium sulphate) and solvent removal under reduced pressure gave the crude acid (347 mg) which was chromatographed (silica gel, 0-10% methanol/dichloromethane) to yield 3-[(5-(2,3-epoxy-5-hydroxy-4-methylhexyl)-3,4-dihydroxytetrahydropyran-2-yl)-methyl]-2-methylbut-3-enoic acid (147 mg, 0.41 mmol, 41%).

i.r. spectrum

$\nu_{max}$ (film), 3600-3100, 2980, 2930, 1710, 1635, 1450, 1380, 1120, 1050, 975, 900 $cm^{-1}$;

$^1H$ nmr

$\delta_H$ ($CD_3OD$) 0.95 (3H, d, J=7Hz, $CH_3$-17), 1.00-1.30 (6H, 2xd, $CH_3$-14, $CH_3$-C2), 1.40 (1H, m, CH-12), 1.75 (2H, m, $CH_2$-9), 1.95 (1H, m, CH-8), 2.20 (1H, dd, J=14,8Hz, CH-4a), 2.62 (1H, bd, J=14Hz, CH-4b), 2.71 (1H, dd, J=7,2Hz, CH-11), 2.80 (1H, dt, J=2,5Hz, CH-10), 3.20-3.40 (obscured by solvent), 3.50-3.90 (7H, m), 5.05 (2H, bs, $CH_2$-15);

$^{13}C$ nmr

$\delta_C$ ($CD_3OD$) 12.3 (C17), 17.0 ($CH_3$-C2), 20.3 (C14), 33.0 (C9), 38.2 (C4), 41.4 (C8), 43.7 (C12), 46.8, 48.0 (C2), 56.9 (C10), 61.4 (C11), 66.2 (C10), 69.9 (C6), 70.7 (C7), 71.6 (C13), 77.4 (C5), 113.1 (C15), 147.4 (C3), 178.3 (C1);

mass spectrum (Chemical ionisation using ammonia)
m/e (relative intensity) 376 (15%, $MNH_4^+$), 359 (100%, $MH^+$), 341 (80), 323 (39), 315 (37), 297 (21), 227 (79).

Example 11

4-(Methylthio)benzyl 2-methylmonate

4-(Methylthio)benzyl chloride (174 mg, 1.01 mmol) was treated with sodium 2-methylmonate (254 mg, 0.67 mmol) in DMF (5 ml) overnight at room temperature and then evaporated *in vacuo*. The residue was taken up in ethyl acetate/water, washed with brine, then dried ($MgSO_4$) and evaporated *in vacuo*. The resulting residue was purified by chromatography (on silica, 0 to 5% methanol in dichloromethane) to yield the title compound 173 mg, 0.35 mmol, 52%); mp 100-101° (from $Et_2O$); $\nu_{max}$(film) 3600 - 3200, 2970, 2920, 1710, 1630, 1605, 1495, 1450, 1375, 1280, 1210, 1090, 1050, 805, 730 $cm^{-1}$ $\lambda_{max}$(EtOH) 259 nm ($\varepsilon_m$ 17,100); $\delta_H$($CDCl_3$) 0.94 (3H, d, J = 7 Hz, $CH_3$-17), 1.24 (3H, Cl, J = 7 Hz, $CH_3$-14), 1.32 (1H, m, CH-12), 1.72 (2H, m, $CH_2$-9), 1.91 (3H, s, $CH_3$-C2), 2.04 (4H, s + m, $CH_3$-15 + CH-8), 2.34 (1H, dd, J = 9, 14 Hz, CH-4a), 2.50 (3H, s, $SCH_3$), 2.58 (1H, dd, J = 14, 2 Hz, CH-4b), 2.69 (1H, dd, J = 9, 1 Hz, CH-11), 2.80 (1H, m, CH-10), 3.4 - 4.0 (6H, m), 5.12 (2H, s, $CH_2$), 7.2 - 7.4 (4H, m); $\delta_C$($CDCl_3$) 12.6 (C17), 15.8 ($SCH_3$), 20.8 (C14), 21.6 (C15), 31.7 (C9), 38.2 (C4), 42.8 (C12), 55.6 (C10), 61.3 (C11), 65.4 (C16), 65.7 ($CH_2$), 69.5 (C6), 70.4 (C7), 71.1 (C13), 76.0 (C5), 124.2 (C2), 126.7, 128.8 (C2', 6' + C3', 5'), 133.1 (C4'), 138.5 (C1'), 144.4 (C3), 169.7 (C1); m/e (relative intensity) 494 ($M^+$, <1%), 227 (4), 138 (11), 137 (100), 125 (9), 69 (13), 55 (9), 43 (17), 41 (14) (Found: 494.2332, $C_{26}H_{38}O_7S$ requires 494.2335).

Example 12

4-Bromobenzyl 2-methylmonate A

4-Bromobenzyl bromide (125 mg, 0.50 mmol) was treated with sodium 2-methylmonate (128 mg, 0.34 mmol) in DMF (5 ml) overnight at room temperature and the solvent evaporated *in vacuo*. The residue was taken up in ethyl acetate/water washed with brine, then dried ($MgSO_4$) and evaporated *in vacuo*. The resulting residue was purified by chromatography on silica (0-5% methanol in dichloromethane) to yield the title compound (29 mg, 0.05 mmol, 16%); $\nu_{max}$(film) 3600 - 3200, 2970, 2930, 1710, 1640, 1490, 1450, 1380, 1280, 1210, 1100, 1010, 910, 805, 730 $cm^{-1}$; $\lambda_{max}$ (EtOH) 227 nm ($\varepsilon_m$ 18,000); $\delta_H$($CDCl_3$) 0.94 (3H, d, J = 7 Hz, $CH_3$-17), 1.22 (3H, d, J = 7 Hz, $CH_3$-14), 1.33 (1H, m, CH-12), 1.74 (2H, m, $CH_2$-9), 1.92 (3H, s, $CH_3$-C2), 2.03 (4H, s + m, CH-8, $CH_3$-15), 2.35 (1H, dd, J = 9, 14 Hz, CH-4a), 2.59 (1H, dd, J = 14, 3 Hz, CH-4b), 2.70 (1H, dd, J = 2, 8 Hz, CH-11), 2.82 (1H, dt, J = 2, 5 Hz, CH-10), 3.4 - 4.0 (6H, m), 5.12 (2H, s, $CH_2$-Ar), 7.25 (2H, d, J = 8 Hz, CH-3', 5'), 7.50 (2H, d, J = 8 HZ, CH-2', 6'); $\delta_C$($CDCl_3$) 12.7 (C17), 15.8 ($CH_3$-C2), 20.8 (C14), 21.6 (C15), 31.7 (C9), 38.2 (C4), 39.5 (C8), 42.8 (C12), 55.6 (C10), 61.3 (C11), 65.2 ($CH_2$), 65.4 (C16), 69.5 (C6), 70.4 (C7), 71.3 (C13), 76.0 (C5), 122.1 (C4'), 124.1 (C2), 129.8 (C2', 6'), 131.7 (C3', 5'), 135.4 (C1'), 144.8 (C3), 169.4 (C1); m/e (relative intensity) 528, 526 ($M^+$, <1%), 227 (45), 171 (96), 169 (100), 125 (38), 69 (63), 45 (41), 43 (83), 41 (67).

Example 13

8-Hydroxyoctyl 2-methylmonate A

8-Hydroxyoctyl bromide (96 mg, 0.50 mmol) was treated with sodium 2-methylmonate (128 mg, 0.34 mmol) in DMF (5 ml) overnight at room temperature and then evaporated *in vacuo*. The residue was taken up in ethylacetate/water, washed with brine, then dried ($MgSO_4$) and evaporated *in vacuo*. The resulting residue was purified by chromatography (on silica, 0 to 5% methanol in dichloromethane) to yield the title compound (40 mg, 0.08 mmol, 24%); $\nu_{max}$ (film) 3600 - 3200, 2930, 2860, 1700, 1630, 1450, 1380, 1280, 1215, 1100, 1055, 730 $cm^{-1}$; $\lambda_{max}$ (EtOH) 226 nm ($\varepsilon_m$ 9,600); $\delta_H(CDCl_3)$ 0.94 (3H, d, J = 7 Hz, $CH_3$-17), 1.22 (3H, d, J = 7 Hz, $CH_3$-14), 1.34 (9H, bs), 1.56 (2H, m), 1.67 (2H, m), 1.73 (2H, m, $CH_2$-9), 1.89 (3H, s, $CH_3$-C2), 2.02 (4H, s + m, $CH_3$-15 + CH-8), 2.33 (1H, dd, J = 14, 8 Hz, CH-4a), 2.57 (1H, dd, J = 14, 3 Hz, CH-4b), 2.72 (1H, dd, J = 8, 2 Hz, CH-11), 2.81 (1H, dt, J = 2, 5 Hz, CH-10), 3.4, 3.55 (2H, m), 3.64 (2H, t, J = 7 Hz, $CH_2$-1'), 3.65 - 4.00 (4H, m), 4.12 (2H, t, J = 7 Hz, $CH_2$-8'); $\delta_C(CDCl_3)$ 12.6 (C17), 15.8 ($CH_3$-C2), 20.7 (C14), 21.5 (C15), 25.7 (C7'), 26.0 (C3'), 28.6 (C2'), 29.1, 29.3 (C4', 5', 6'), 31.7 (C9), 38.0 (C4), 39.6 (C9), 42.8 (C12), 55.7 (C10), 61.2 (C11), 62.8 (C8'), 64.4 (C1'), 65.4 (C16), 69.5 (C6), 70.4 (C7), 71.1 (C13), 76.1 (C6), 124.6 (C2), 143.2 (C3), 170.3 (C1); m/e (relative intensity) 486 ($M^+$, 1%), 227 (37), 125 (48), 97 (44), 96 (83), 69 (100), 55 (88), 45 (68), 43 (83) (Found: 486.3157, $C_{26}H_{46}O_8$ requires 486.3193).

## Example 14

### 3-Methyl-5-isoxazolylmethyl 2-methylmonate A

A solution of 5-hydroxymethyl-3-methylisoxazole (1.13g, 10 mmol) and triphenylphosphine (2.62 g, 10 mmol) in carbon tetrachloride (20 ml) was heated at reflux for 3½ h, then the solvent removed *in vacuo*. The residue was taken up in ether, the triphenylphosphine oxide filtered off then the residue purified by flash chromatography (20% ether in hexane) to give 5-chloromethyl-3-methylisoxazole (820 mg, 6.23 mmol, 62%); $\nu_{max}$(film) 3130, 2970, 2930, 1610, 1450 - 1410, 1285, 1265, 1145, 1130, 1020, 1005, 920, 870, 810, 740, 730 $cm^{-1}$; $\delta_H(CDCl_3)$ 2.25 (3H, s, $CH_3$-3), 4.55 (2H, s, $CH_2$-Cl), 6.15 (1H, s, CH-4).

3-Methyl-5-isoxazolylmethyl chloride (66 mg, 0.5 mmol) was treated with sodium 2-methylmonate (114 mg, 0.3 mmol) in DMF (5 ml) overnight at room temperature and then evaporated *in vacuo*. The residue was taken up in ethyl acetate/water, washed with brine, then dried ($MgSO_4$) and evaporated *in vacuo*. The resulting residue was purified by chromatography (on silica, 0 to 5% methanol in dichloromethane) to yield the title compound (107.3 mg, 0.24 mmol, 79%); mp 104-104.5$^o$ (from $Et_2O$); $\nu_{max}$(film) 3600 - 3200, 2970, 2930, 1715, 1615, 1450, 1380, 1280, 1210, 1100, 1050, 910, 730 $cm^{-1}$; $\lambda_{max}$(EtOH) 224 nm ($\varepsilon_m$13,100); $\delta_H(CDCl_3)$ 0.93 (3H, d, J = 7 Hz, $CH_3$-17), 1.21 (3H, d, J = 7 Hz, $CH_3$-14), 1.33 (1H, m, CH-12), 1.72 (2H, t, J = 5 Hz, $CH_2$-9), 1.90 (3H, s, $CH_3$-C2), 2.00 (1H, m, CH-8), 2.05 (3H, s, $CH_3$-15), 2.30 (3H, s, $CH_3$-het), 2.35 (1H, dd, J=14, 8Hz, CH-4a), 2.55-2.85 (3H, m, CH-10, 11, 4b),

3.4-4.0 (6H, m), 5.20 (2H, s, $CH_2$), 6.50 (1H, s, het-H); $\delta_C$ ($CDCl_3$) 11.3 (C5'), 12.6 (C17), 15.7 ($CH_3$-$C_2$), 20.8 (C14), 21.7 (C15), 31.7 (C9), 38.5 (C4), 39.6 (C8), 42.8 (C12), 55.6 (C10), 56.2 (C1'), 61.3 (C11), 65.4 (C16), 69.4 (C6), 70.4 (C7), 71.2 (C13), 76.0 (C5), 104.5 (C3'), 123.3 (C2), 146.7 (C3), 159.9 (C4'), 167.0 (C2'), 168.7 (C1); m/e (relative intensity, CI, $NH_3$) 454 (30%, $MH^+$), 341 (100), 323 (42), 227 (18), 114 (68), 100 (48), 98 (26), 97 (31), 84 (20) (Found: C, 60.73; H, 7.57; N, 3.14%. $C_{23}H_{35}NO_8$ requires C, 60.91; H, 7 78; N, 3.09%).

## Biological Data

### a) Mycoplasma

The activity of the compounds of the Examples against various mycoplasmal organisms was assayed *in vitro* in Friis broth solidified with 0.9% agarose and inoculated with $10^3$ to $10^5$ C.F.U. The minimum inhibitory concentrations (MIC's) were determined after incubation for 6 days at 37°C and are shown in Table I.

### b) Veterinary Bacteria

The activity of the compounds of the Examples against various veterinarily important bacteria, was assayed in vitro using two-fold serial dilutions in Diagnostic Sensitivity Test Agar inoculated with $10^4$ organisms. The MIC's were determined after incubation for 18 hours at 37°C and are shown in Table 2.

### c) Human Bacteria

The activity of the compounds of the Examples against various bacteria which are important in diseases of humans, was assayed *in vitro* using serial dilutions in nurient agar with 5% chocolated horse blood. The MIC's were determined after incubation for 18 hours at 37°C and are shown in Table 3.

Table 1

MIC's (µg/ml) against Mycoplasma

| Organism | Compound of Example No. | | | | |
|---|---|---|---|---|---|
| | 1 | 2* | 3* | 4 | 6 |
| M. suipneumoniae NB12 | 1.0 | 5.0 | 0.05 | 0.25 | 2.5 |
| M. suipneumoniae JF 435 | 1.0 | 10 | 0.1 | 0.25 | 5.0 |
| M. suipneumoniae HK(2) | 1.0 | 10 | 0.1 | 0.25 | 10.0 |
| M. suipneumoniae Str. 11 | 1.0 | 5.0 | 0.05 | 0.1 | 2.5 |
| M. suipneumoniae J2206/183[b] | 2.5 | 5.0 | 0.1 | 0.25 | 10.0 |
| M. suipneumoniae MS 16 | 0.5 | 5.0 | 0.025 | 0.1 | 2.5 |
| M. suipneumoniae PW/C/210 | 0.5 | 5.0 | 0.025 | 0.1 | 2.5 |
| M. suipneumoniae LABER | 1.0 | 5.0 | 0.05 | 0.1 | 2.5 |
| M. suipneumoniae UCD 1 | 2.5 | 10 | 0.05 | 0.25 | 10.0 |
| M. suipneumoniae TAM 6N | 2.5 | 10 | 0.1 | 0.25 | 10.0 |
| M. suipneumoniae ATCC 25095 | 1.0 | 5.0 | 0.025 | 0.25 | 2.5 |
| M. suipneumoniae NCTC 10110 | 1.0 | 5.0 | 0.05 | 0.25 | 10.0 |
| M. hyorhinis ATCC 23234 | 0.5 | 5.0 | 0.025 | 0.1 | 1.0 |
| M. hyorhinis ATCC 25021 | 0.5 | 5.0 | 0.025 | 0.1 | 1.0 |
| M. hyosynoviae ATCC 25591 | 1.0 | 5.0 | 0.25 | 0.25 | 1.0 |
| M. bovis NCTC 10131 | 0.05 | 0.25 | 0.025 | 0.025 | 0.1 |
| M. bovigenitalium ATCC 14173 | 0.05 | 0.5 | 0.025 | 0.05 | 0.25 |
| M. dispar NCTC 10125 | 0.25 | 1.0 | <0.01 | 0.05 | 2.5 |
| M. gallisepticum S6 | >10 | >10 | 5.0 | 5.0 | >10.0 |
| M. pneumoniae ATCC 15492 | 10 | NG | 2.5 | 5.0 | 10.0 |

* Results for the E-isomer. The Z-isomer was inactive at the concentrations tested.

NG - no growth

Table 2

MIC's (μg/ml) against Veterinary Bacteria

| Organism | Compound of Example No. | | | | |
|---|---|---|---|---|---|
| | 1 | 2* | 3* | 4 | 6 |
| E. coli NCTC 10418 | >80 | >80 | >80 | >80 | >80 |
| E. coli E1 | >80 | >80 | >80 | >80 | >80 |
| S. dublin S7 | >80 | >80 | >80 | >80 | >80 |
| S. typhimurium S18 | >80 | >80 | >80 | >80 | >80 |
| Bord. bronchiseptica B08 | >80 | >80 | 80 | >80 | 40 |
| Bord. bronchiseptica B09 | 80 | 40 | 80 | 40 | 5.0 |
| Past. multocida PA1 | 2.5 | 10 | 2.5 | 5.0 | 5.0 |
| Past. multocida PA2 | 1.25 | 5 | 1.25 | 2.5 | 5.0 |
| Past. haemolytica PA5 | 20 | 40 | 10 | 10 | 80 |
| Erysipelothrix rhusiopathiae NCTC 8163 | 80 | >80 | 40 | 80 | >80 |
| Corynebacterium pyogenes CY1 | >80 | >80 | >80 | 80 | >80 |
| Staph. aureus B4 (pen. resistant) | 0.625 | 5 | 1.25 | 1.25 | 1.25 |
| Staph. aureus 152 (pen. sens) | 0.625 | 5 | 1.25 | 1.25 | 2.5 |
| Staph. aureus Oxford | 0.625 | 5 | 1.25 | 1.25 | 0.625 |
| Strep. suis (group D) SPS11 | >80 | >80 | 10 | 20 | 10 |
| Strep. uberis SPU1 | 1.25 | 20 | 0.156 | 0.156 | 0.312 |
| Strep. dysgalactiae SPD1 | 5 | 40 | 0.312 | 1.25 | 0.625 |
| Strep. agalactiae SPA1 | 5 | 80 | 1.25 | 1.25 | NG |
| B. subtilis ATCC 6633 | NT | NT | NT | NT | NT |

* Results for the E-isomer. The Z-isomer was inactive at the concentrations tested.

NG - no growth
NT - not tested

Table 1

MIC's (µg/ml) against Mycoplasma

| Organism | Compound of Example No. | | | | |
|---|---|---|---|---|---|
| | 7 | 11 | 12 | 13 | 14 |
| M. suipneumoniae NB12 | 10 | 1.0 | 5.0 | 1.0 | 0.5 |
| M. suipneumoniae JF 435 | >10 | 2.5 | 10 | 1.0 | 0.5 |
| M. suipneumoniae HK(2) | >10 | 2.5 | 10 | 1.0 | 0.5 |
| M. suipneumoniae Str. 11 | 5 | 1.0 | 5.0 | 0.5 | 0.25 |
| M. suipneumoniae J2206/183[b] | >10 | 2.5 | 10 | 1.0 | 0.5 |
| M. suipneumoniae MS 16 | 5 | 1.0 | 5.0 | 0.5 | 0.25 |
| M. suipneumoniae PW/C/210 | 5 | 1.0 | 5.0 | 0.5 | 0.25 |
| M. suipneumoniae LABER | 5 | 1.0 | 5.0 | 0.5 | 0.25 |
| M. suipneumoniae UCD 1 | >10 | 2.5 | 10 | 1.0 | 0.5 |
| M. suipneumoniae TAM 6N | >10 | 2.5 | 10 | 1.0 | 1.0 |
| M. suipneumoniae ATCC 25095 | 10 | 2.5 | 5.0 | 0.5 | 0.5 |
| M. suipneumoniae NCTC 10110 | 10 | 2.5 | 10 | 1.0 | 0.5 |
| M. hyorhinis ATCC 23234 | 2.5 | 1.0 | 2.5 | 0.5 | 0.25 |
| M. hyorhinis ATCC 25021 | 2.5 | 0.5 | 2.5 | 0.5 | 0.25 |
| M. hyosynoviae ATCC 25591 | 1.0 | 0.25 | 2.5 | 0.1 | 0.1 |
| M. bovis NCTC 10131 | 0.05 | 0.05 | 0.1 | ≤0.01 | ≤0.01 |
| M. bovigenitalium ATCC 14173 | 0.5 | 0.1 | 0.25 | 0.05 | 0.025 |
| M. dispar NCTC 10125 | 2.5 | 0.5 | 2.5 | 0.1 | 0.1 |
| M. gallisepticum S6 | >10 | 10 | >10 | 5.0 | 1.0 |
| M. pneumoniae ATCC 15492 | >10 | 5.0 | 10 | 1.0 | 1.0 |

* Results for the E-isomer. The Z-isomer was inactive at the concentrations tested.

NG - no growth

Table 2

MIC's (µg/ml) against Veterinary Bacteria

| Organism | Compound of Example No. | | | | |
|---|---|---|---|---|---|
| | 7 | 11 | 12 | 13 | 14 |
| E. coli NCTC 10418 | >80 | >80 | >80 | >80 | >80 |
| E. coli E1 | >80 | >80 | >80 | >80 | >80 |
| S. dublin S7 | >80 | >80 | >80 | >80 | >80 |
| S. typhimurium S18 | >80 | >80 | >80 | >80 | >80 |
| Bord. bronchiseptica B08 | >80 | 80 | 40 | >80 | 80 |
| Bord. bronchiseptica B09 | 40 | 10 | 5.0 | 40 | 10 |
| Past. multocida PA1 | 1.25 | 10 | 5.0 | 2.5 | 1.25 |
| Past. multocida PA2 | 2.5 | 0.156 | 5.0 | 5.0 | <0.039 |
| Past. haemolytica PA5 | 20 | 20 | 10 | 20 | 5.0 |
| Erysipelothrix rhusiopathiae NCTC 8163 | >80 | >80 | 80 | 80 | 80 |
| Corynebacterium pyogenes CY1 | >80 | >80 | >80 | >80 | >80 |
| Staph. aureus B4 (pen. resistant) | 1.25 | 0.156 | 0.312 | 2.5 | 1.25 |
| Staph. aureus 152 (pen. sens) | 1.25 | 0.156 | 0.625 | 1.3 | 0.312 |
| Staph. aureus Oxford | 1.25 | 0.156 | 0.625 | 1.3 | 0.625 |
| Strep. suis (group D) SPS11 | 80 | 5.0 | 40 | 80 | 5.0 |
| Strep. uberis SPU1 | 0.625 | <0.039 | 1.25 | 0.156 | 0.156 |
| Strep. dysgalactiae SPD1 | 1.25 | 0.312 | 0.156 | 0.156 | 0.312 |
| Strep. agalactiae SPA1 | 80 | 0.625 | 40 | 80 | 0.625 |
| B. subtilis ATCC 6633 | NT | NT | NT | NT | NT |

* Results for the E-isomer. The Z-isomer was inactive at the concentrations tested.

NG - no growth
NT - not tested

Table 3

MIC's (µg/ml) against Human Bacteria

| Organism | Compound of Example No. | | | | |
|---|---|---|---|---|---|
| | 1 | 2* | 3* | 4 | 6 |
| E. coli NCTC 10418 | >100 | >100 | >100 | >50 | >100 |
| E. coli ESS | 5.0 | 25 | 1.0 | 2.5 | 2.5 |
| P. mirabilis 889 | >100 | >100 | >100 | >50 | >100 |
| K. aerogenes A | >100 | >100 | >100 | >50 | >100 |
| Ps. aeruginosa NCTC 10662 | >100 | >100 | >100 | >50 | >100 |
| Pasteurella multocida 1633 | 5.0 | 10 | 5.0 | 5.0 | 2.5 |
| Haemophilus influenzae Q1 | 0.5 | 1.0 | 0.5 | 0.5 | 0.2 |
| Haemophilus influenzae Wy21 | 1.0 | NG | NG | 0.2 | 0.1 |
| Neisseria catarrhalis 1502 | 2.5 | 2.5 | 0.5 | 1.2 | 2.5 |
| Bacillus subtilis 6633 | 5.0 | 5.0 | 0.5 | 1.2 | 0.5 |
| Corynebacterium xerosis 9755 | >100 | >100 | >100 | > 50 | >100 |
| Sarcina lutea 8340 | >100 | >100 | >100 | > 50 | >100 |
| Staph. aureus Oxford | 1.0 | 5.0 | 2.5 | 1.2 | 1.0 |
| Staph. aureus Russell | 1.0 | 5.0 | 2.5 | 5.0 | 2.5 |
| Staph. aureus W2827 | 1.0 | 5.0 | 2.5 | 5.0 | 2.5 |
| Strep. faecalis I | >100 | >100 | 50 | 50 | >100 |
| Strep. pyogenes R80/421-A | 2.5 | 5.0 | 0.5 | NG | NG |
| Strep. pyogenes B2788 | 10.0 | NG | 1.0 | 2.5 | 5.0 |
| Strep. pyogenes 64/848-C | 10.0 | 25 | 0.5 | NG | NG |
| Strep. pneumoniae CN33 | 5.0 | NG | 0.5 | 2.5 | 5.0 |

* Results for the E-isomer. The Z-isomer was inactive at the concentrations tested.

NG - no growth

Table 3

MIC's (μg/ml) against Human Bacteria

| Organism | Compound of Example No. | | | | |
|---|---|---|---|---|---|
| | 7 | 11 | 12 | 13 | 14 |
| E. coli NCTC 10418 | >64 | >128 | >128 | >128 | >128 |
| E. coli ESS | 32 | 8.0 | 32 | 4.0 | 2.0 |
| P. mirabilis 889 | >64 | >128 | >128 | >128 | >128 |
| K. aerogenes A | >64 | >128 | >128 | >128 | >128 |
| Ps. aeruginosa NCTC 10662 | >64 | >128 | >128 | >128 | >128 |
| Pasteurella multocida 1633 | 2.0 | 8.0 | 32 | 2.0 | 1.0 |
| Haemophilus influenzae Q1 | 0.25 | 1.0 | 2.0 | 1.0 | 0.25 |
| Haemophilus influenzae Wy21 | 0.25 | 1.0 | 2.0 | 1.0 | 0.25 |
| Neisseria catarrhalis 1502 | 4.0 | 1.0 | 4.0 | 2.0 | 0.25 |
| Bacillus subtilis 6633 | 0.5 | 1.0 | 0.5 | 0.25 | 0.25 |
| Corynebacterium xerosis 9755 | >128 | >128 | >128 | >128 | >128 |
| Sarcina lutea 8340 | >128 | >128 | >128 | >128 | >128 |
| Staph. aureus Oxford | 2.0 | 1.0 | 2.0 | 1.0 | 1.0 |
| Staph. aureus Russell | 2.0 | 1.0 | 2.0 | 1.0 | 1.0 |
| Staph. aureus W2827 | 2.0 | 1.0 | 2.0 | 1.0 | 1.0 |
| Strep. faecalis I | >128 | >128 | >128 | >128 | 64 |
| Strep. pyogenes R80/421-A | 8.0 | 2.0 | 4.0 | 4.0 | 1.0 |
| Strep. pyogenes B2788 | 8.0 | 2.0 | 4.0 | 4.0 | 1.0 |
| Strep. pyogenes 64/848-C | - | 1.0 | - | - | - |
| Strep. pneumoniae CN33 | 5.0 | 2.0 | 4.0 | 4.0 | 1.0 |

* Results for the E-isomer. The Z-isomer was inactive at the concentrations tested.

NG - no growth

CLAIMS

1. An acid of formula (I):

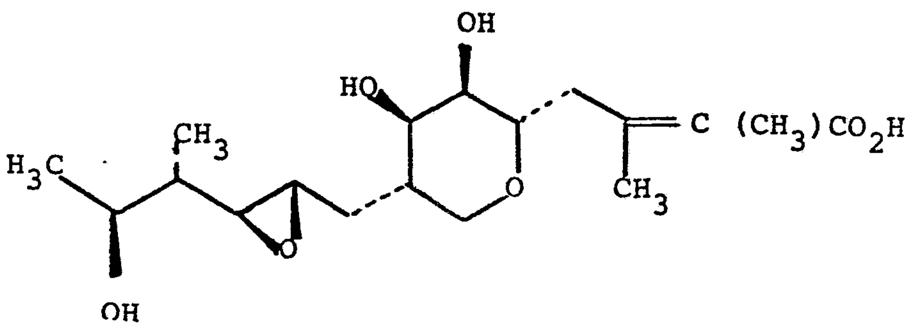

(I)

or a salt or ester thereof.

2. An acid of formula (I) according to claim 1 or an aluminium, alkaline earth metal, alkali metal, ammonium or substituted ammonium salt thereof.

3. An optionally substituted alkyl, cycloalkyl, alkenyl, alkynyl or aralkyl ester, of an acid of formula (I) according to claim 1 .

4. An ester of formula (IA):

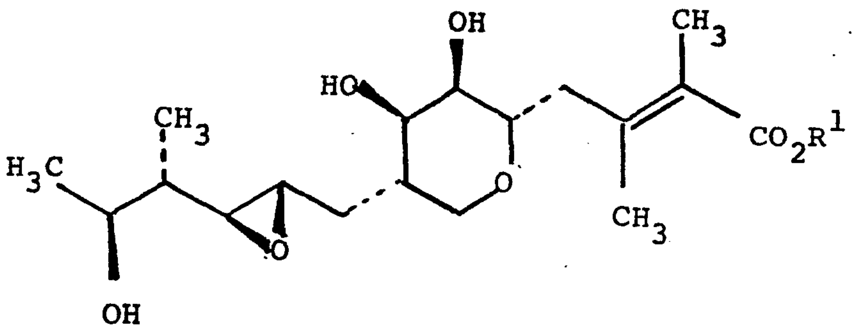

(IA)

wherein $R^1$ is $C_{1-20}$ alkyl, $C_{3-7}$ cycloalkyl, $C_{2-8}$ alkenyl, $C_{2-8}$ alkynyl each of which may be optionally substituted.

5. An ester according to claim 4 wherein $R^1$ is selected from alkyl, cycloalkyl, alkenyl and alkynyl groups optionally substituted by halogen, carboxy, $C_{1-6}$ alkoxycarbonyl, carbamoyl, mono- or di-$(C_{1-6})$alkylcarbamoyl, sulphamoyl, mono- and di-$(C_{1-6})$alkylsulphamoyl, amino, mono- and di-$(C_{1-6})$alkylamino, $C_{1-6}$ acylamino, ureido, $C_{1-6}$ alkoxycarbonylamino, 2,2,2-trichloroethoxy-carbonylamino, optionally substituted phenyl, heterocyclyl, hydroxy, $C_{1-6}$ alkoxy, oxo, aroyl, 2-thenoyl, $C_{1-6}$ alkylthio, $C_{1-6}$ alkanesulphinyl, $C_{1-6}$ alkanesulphonyl, hydroxyimino, hydrazono, benzohydroximoyl, 2-thiophenecarbohydroximoyl.

6. An ester according to claim 4 wherein $R^1$ is phenyl optionally substituted by halogen, $C_{1-6}$ alkyl, $C_{1-6}$ alkoxy, hydroxy, carboxy, $C_{1-6}$ alkoxycarbonyl, carbamoyl, mono- or di-$(C_{1-6})$alkylcarbamoyl, sulphamoyl, mono- and di-$(C_{1-6})$sulphamoyl, cyano, nitro, amino, mono- and di-$(C_{1-6})$alkylamino, $C_{1-6}$ acylamino, ureido, $C_{1-6}$ alkoxycarbonylamino,

2,2,2-trichloroethoxy-carbonylamino, $C_{1-6}$ alkanoyl, $C_{1-6}$ alkylthio, $C_{1-6}$ alkanesulphinyl or $C_{1-6}$ alkanesulphonyl.

7. A process for producing a compound of formula (I) which process comprises:

(a) methylation of a compound of formula (II):

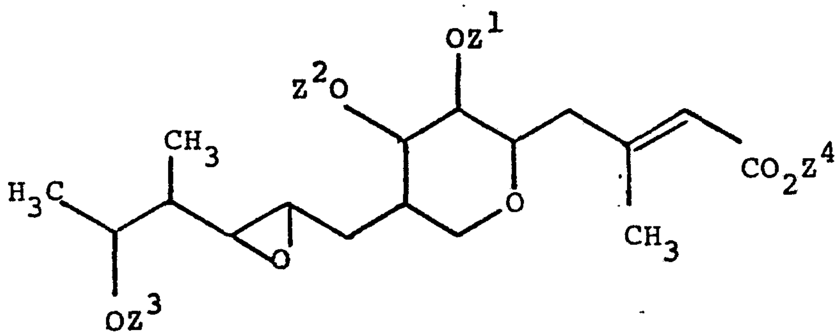

(II)

wherein $Z^1$, $Z^2$ and $Z^3$ are hydrogen or hydroxyl-protecting groups;

and $Z^4$ is hydrogen, a salt-forming cation, or ester-forming radical or a carboxyl-protecting group,

or (b) reacting a compound of formula (III):

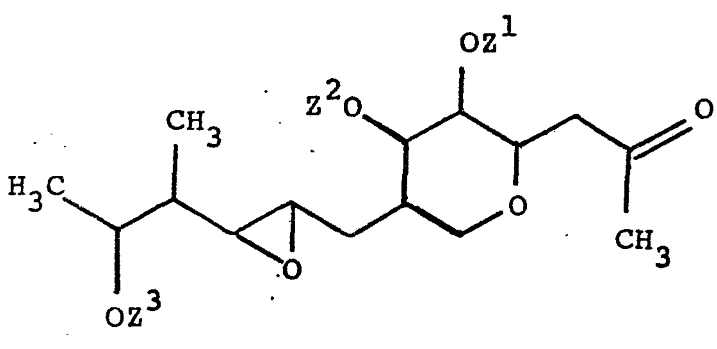

(III)

wherein $Z^1$, $Z^2$ and $Z^3$ are hydroxyl-protecting groups with a compound of formula (IV):

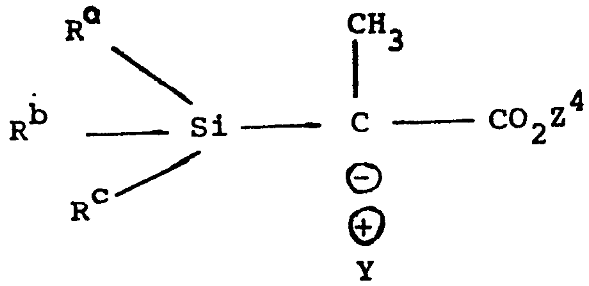

(IV)

wherein $Z^4$ is as defined with respect to formula (II),

$R^a$, $R^b$ and $R^c$ are the same or different and each is lower alkyl, aryl or aralkyl; and $Y^+$ is a counter-ion;

or (c) converting a compound of formula (V):

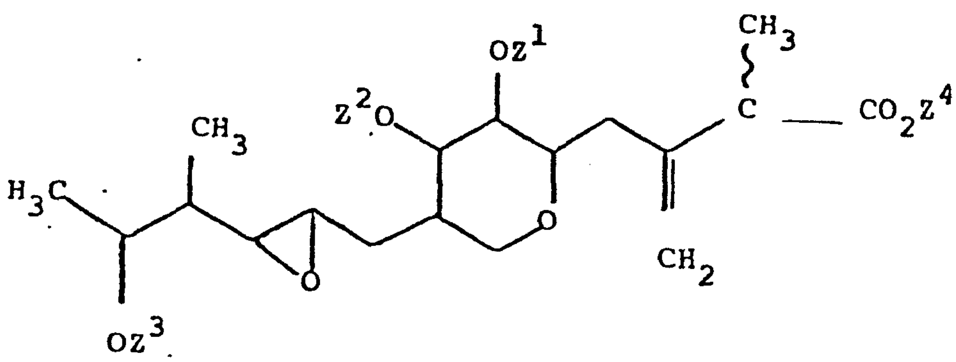

wherein $Z^4$ is as defined with respect to formula (II),

and $Z^1$, $Z^2$ and $Z^3$ are hydrogen or hydroxyl-protecting groups,

into a compound of formula (I) or a protected derivative thereof;

and, if necessary, isomerising a Z isomer so obtained to produce the corresponding E isomer, and, if necessary, removing any

hydroxyl-protecting groups and/or carboxyl protecting groups to form the desired compound of formula (I),

and optionally esterifying an acid or salt of formula (I) and de- or trans-esterifying an ester of formula (I).

8. A process according to claim 7 comprising converting a compound of formula (V) to a compound of formula (I) using an alkali metal alkoxide, in a suitable solvent at -78°C to 20°C, or an amine base at up to 100°C.

9. A compound of formula (V),

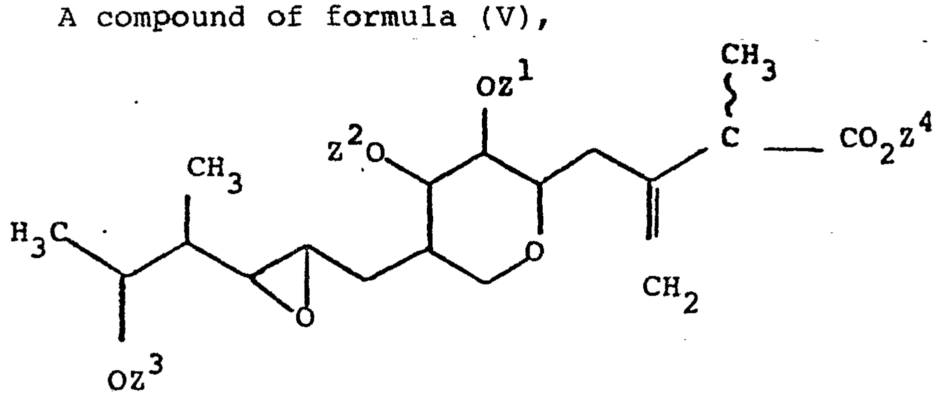

(V)

wherein $Z^4$ is hydrogen, a salt-forming cation, or ester-forming radical or a carboxyl-protecting group,
and $Z^1$, $Z^2$ and $Z^3$ are hydrogen or hydroxyl-protectig groups.

10. A pharmaceutical or veterinary composition which comprises an ester of an acid of formula (I) together with a pharmaceutically or veterinarily acceptable carrier or excipient.